Europäisches Patentamt

European Patent Office

Office européen des brevets

(19)

(11) Veröffentlichungsnummer : **0 057 839**
**B1**

(12) **EUROPÄISCHE PATENTSCHRIFT**

(45) Veröffentlichungstag der Patentschrift :
28.08.85

(21) Anmeldenummer : 82100430.6

(22) Anmeldetag : 22.01.82

(51) Int. Cl.⁴ : **C 08 L 75/04,** C 08 G 18/08,
C 08 G 18/42, C 08 G 18/48,
C 08 J 3/02, B 01 J 13/00,
A 01 N 25/18, A 61 K 7/32,
A 61 K 7/40, B 27 K 3/14,
B 29 C 33/40

(54) Gegebenenfalls wirkstoffhaltige Gelmassen auf Basis einer Polyurethanmatrix und höhermolekularen Polyolen, ein Verfahren zu ihrer Herstellung, sowie ihre Verwendung.

(30) Priorität : 03.02.81 DE 3103499
03.02.81 DE 3103500

(43) Veröffentlichungstag der Anmeldung :
18.08.82 Patentblatt 82/33

(45) Bekanntmachung des Hinweises auf die Patenterteilung : 28.08.85 Patentblatt 85/35

(84) Benannte Vertragsstaaten :
AT BE CH DE FR GB IT LI NL SE

(56) Entgegenhaltungen :
EP-A- 0 016 652
DE-A- 2 347 299
DE-A- 2 754 249
DE-C- 1 168 075

(73) Patentinhaber : BAYER AG
Konzernverwaltung RP Patentabteilung
D-5090 Leverkusen 1 Bayerwerk (DE)

(72) Erfinder : Schäpel, Dietmar, Dr,
Johanniterstrasse 15
D-5000 Köln 80 (DE)

## 0 057 839

**Beschreibung**

Die Erfindung betrifft wasserfreie Gelmassen, gegebenenfalls wirkstoffhaltige Gelmassen mit Depotwirkung, bestehend aus einer Polyurethanmatrix und höhermolekularen Polyolen als Dispersionsmittel und gegebenenfalls Wirkstoffen, sowie ein Verfahren zur Herstellung der Gelmassen, gegebenenfalls in Anwesenheit der Wirkstoffe. Als Wirkstoffe können Biozide, Pharmazeutika, etherische Öle, Duftstoffe, Farben, reinigungsmittel, Alterungsschutzmittel, Gleit- und Antistatikmittel und andere eingesetzt werden. Die Gelmassen können als Abform- oder Eingußmassen oder als wirkstoffhaltige Formteile mit Depotwirkung der Wirkstoffe Verwendung finden.

Gele auf wäßriger Basis werden seit Jahren in vielen technischen Bereichen verwendet (s. z. B. R.L. Whistler, Industrial Gums, Academic Press, InC., New York, 1973 und De-AS 2 347 299). Eine besonders interessante Eigenschaft der Gele besteht darin, daß sie eine hohe Abformgenauigkeit aufweisen. Dies wird dazu genutzt, um Formkörper zu doublieren. Dabei wird der abzuformende Körper mit der gelbildenden Masse umgossen. Nach der Gelbildung wird der Formkörper entnommen. Man erhält eine Gelform, deren Hohlraum dem Volumen des Formkörpers entspricht. Als Doubliermasse wird z. B. im Dentalbereich ein Agar-Agar-Gel verwendet. Solche Massen weisen jedoch eine Reihe von Nachteilen auf :

a) Die Gelierung dauert lange und muß unter bestimmten Verfahrensbedingungen erfolgen,

b) die Elastizität des Gels ist für die entformung von dünnen Stegen und Hinterschnitten nicht hoch genug und

c) die Dimensionsstabilität ist nicht befriedigend ; bei offener Lagerung der Gelform tritt bereits nach sehr kurzer Zeit infolge der Wasserverdunstung eine Veränderung der Proportionen ein.

Weiterhin sind wasserfreie Abformmassen, z. B. auf Silikonbasis, bekannt. Sie werden hergestellt, indem man ein Vorpolymerisat mit einer geringen Menge an Vernetzungsmittel vermischt. Das abzuformende Modell wird mit dieser Reaktionsmischung umgossen und nach der Aushärtung der Mischung entnommen. Man erhält eine Form mit einem Hohlraum, in der dann Abgüsse des Modells hergestellt werden können. Wasserfreie Abformmassen besitzen jedoch folgende Nachteile :

a) Zu hohe Viskosität für die Abformung sehr feiner Vertiefungen und Hinterschnitte in der Oberfläche des Models und

b) zu lange Reaktionszeiten ; bei der Verkürzung der Reaktionszeit durch Erhöhung des Vernetzungsmittanteils erfolgt eine zu starke Schrumpfung der Form.

Wirkstoffe enthaltende Gelmassen mit Depotwirkung, bei denen die Wirkstoffe über einen Zeitraum von Wochen bis Monaten an das umgebende Medium abgegeben werden, sind beispielsweise aus den US-Patentschriften 3 822 238 und 3 975 350 bekannt. Weiterhin ist es aus der DE-OS 25 21 265 bekannt, wasser- und/oder alkoholhaltige Polyurethan-Polyharnstoffgele in Gegenwart von Duftstoffen herzustellen. Hierbei werden Trägermaterialien auf Basis Wasser enthaltender Gele beschrieben, die die vielfältigsten Agentien enthalten können, z. B. Pharmazeutika, Biozide oder Duftstoffe. Derartige Wassergele haben jedoch den Nachteil, daß viele Agentien, z. B. Biozide, durch die Anwesenheit des Wassers relativ schnell zersetzt werden können und somit die Zeit der Wirksamkeit dieser Gele, d. h. der Depoteffekt, stark verkürzt wird. Weiterhin ist bekannt, Wirkstoffe massiven und/oder geschäumten hochmolekularen Polyurethanen zu inkorporieren (CH-PS 289 915). Derartige hochmolekulare Polyurethane haben jedoch den Nachteil, daß ein hoher Anteil der inkorporierten flüssigen Agentien in Folge des durchgängig hochmolekularen Aufbaus und/oder zu hohem Hartsegmentanteil im Polyurethan verbleibt und damit für die Depotwirkung verloren ist. Feste aktive Agentien können nur sehr begrenzt eingesetzt werden ; nicht-flüchtige Festsubstanzen wandern nicht heraus und leichflüchtige feste Agentien können nur für sehr kurze Zeit und in sehr geringer Menge herausdiffundieren.

Es wurden nun neuartige Gele auf Basis von Polyolen gefunden, die eine hohe Abformgenauigkeit aufweisen, ohne mit den genannten Nachteilen behaftet zu sein. Die Gele werden erhalten, indem man ein oder mehrere höherfunktionelle, höhermolekulare Polyole in Gegenwart von Katalysatoren und gegebenenfalls Füll- und Zusatzstoffen mit einer solchen Menge an organischen Di- und/oder Polyisocyanaten umsetzt, daß eine Isocyanatkennzahl von etwa 15-60 resultiert. Unter « Isocyanatkennzahl » soll im folgenden das Äquivalenzverhältnis (NCO/OH) × 100 verstanden werden.

Wie gefunden wurde, entstehen nur dann erfindungsgemäße, elastische Gele, die aus einer kovalent vernetzten Polyurethanmatrix und einem oder mehreren darin fest (d. h. ohne die Gefahr eines störenden Ausschwitzens) gebundenen Polyolen aufgebaut sind, wenn die miteinander reagierenden Isocyanat- bzw. Polyolkomponenten eine gewisse Mindestfunktionalität aufweisen und wenn das bzw. die Polyole im wesentlichen frei von Anteilen mit einer OH-Zahl von mehr als 112 bzw. einem Molekulargewicht unterhalb von 800, vorzugsweise unterhalb von 1 000 sind.

Es wurde nun auch überraschend gefunden, daß man Gelmassen mit verbesserter Depotwirkung, gleichmäßiger Wirkstoffabgabe, hoher Wirkstoffkonzentration, guter Stabilität der Wirkstoffzusätze und guter Migrationsfähigkeit der Wirkstoffe erhält, wenn man Wirkstoffe als Zusatzstoffe bei der PUbildenden Reaktion in höhermolekularen Polyolen löst bzw. dispergiert und Di- und/oder Polyisocyanate, sowie Katalysatoren und gegebenenfalls übliche Zusatzstoffe zumischt, wobei die noch zu nennenden Voraussetzungen einzuhalten sind.

2

Die Vorteile der neuartigen Gelmassen liegen darin, daß in der nur teilweise vernetzten Polyurethanmatrix ein hoher Anteil an höhermolekularen Polyolen vorliefgt, welcher die Migration und Abgabe der Wirkstoffe nach außen ermöglicht und steuert.

Die gegebenenfalls wirkstoffhaltigen Gele werden erhalten, indem man ein oder mehrere höherfunktionelle, höhermolekulare Polyole, gegebenenfalls in Gegenwart der Wirkstoffe und gegebenenfalls in Gegenwart von Katalysatoren und üblichen Füll- und Zusatzstoffen für Polyurethane, mit einer solchen Menge an organischen Di- und/oder Polyisocyanaten umsetzt, daß eine Isocyanatkennzahl von etwa 15 bis 60, vorzugsweise 20 bis 55, besonders bevorzugt 25 bis 45, resultiert. Unter « Isocyanatkennzahl » soll im folgenden das Äquivalenzverhältnis (NCO/OH) × 100 verstanden werden.

Wie gefunden wurde, entstehen nur dann erfindungsgemäße, elastische und hinreichend dimensionsstabile Gele, die aus einer kovalent vernetzten Polyurethanmatrix und einem oder mehreren darin fest (d. h. ohne die Gefahr eines störenden Ausschwitzens) gebundenen Polyolen aufgebaut sind, wenn die miteinander reagierenden Isocyanat- bzw. Polyolkomponenten eine gewisse Mindestfunktionalität aufweisen, polyfunktionell sind, und wenn die Polyole im wesentlichen frei von Anteilen mit einem Molekulargewicht unterhalb von 800, vorzugsweise unterhalb von 1 000 sind.

Gegenstand der Erfindung sind somit wasserfreie, gegebenenfalls wirkstoffhaltige Gele bestehend aus

(1) 15-62 Gew.-%, bevorzugt 20-57 Gew.-%, besonders bevorzugt 25-47 Gew.-%, bezogen auf die Summe aus (1) und (2), einer hochmolekularen Matrix und

(2) 85-38 Gew.-%, bevorzugt 80-43 Gew.-%, besonders bevorzugt 75-53 Gew.-%, bezogen auf die Summe aus (1) und (2), eines in der Matrix durch Nebenvalenzkräfte fest gebundenen flüssigen Dispersionsmittels, sowie gegebenenfalls

(3) 0-100 Gew.-%, bezogen auf die Summe aus (1) und (2), an Füll- und/oder Zusatzstoffen, sowie gegebenenfalls Katalysatoren für die polyurethanbildende Reaktion, welche dadurch gekennzeichnet sind, daß

a) die hochmolekulare Matrix ein kovalent vernetztes Polyurethan ist und

b) das flüssige Dispersionsmittel aus einer oder mehreren Polyhydroxylverbindungen mit einem Molekulargewicht zwischen 1 000 und 12 000, vorzugsweise zwischen 1 700 und 6 000, und einer OH-Zahl zwischen 20 und 112, vorzugsweise zwischen 28 und 84, besonders bevorzugt zwischen 30 und 56, besteht, wobei das Dispersionsmittel im wesentlichen keine Hydroxylverbindungen mit einem Molekulargewicht unter 800, worzugsweise keine unter 1 000, enthält, und wobei das Produkt der Funktionalitäten der polyurethan bildenden Komponenten mindestens 52 beträgt und die Isocyanatkennzahl zwischen 15 und 60 liegt.

gegebenenfalls 0,1 bis 50 Gew.-%, vorzugsweise 0,5 bis 35 Gew.-%, besonders bevorzugt 0,75 bis 25 Gew.-%, an Wirkstoffen als Zusatzstoffe in der wirkstoffhaltigen Gelmasse enthalten sind.

Bevorzugt sind solche Gele obiger Zusammensetzung, dadurch gekennzeichnet, daß sie aus 20 bis 57 Gew.-% der hochmolekularen Matrix und 80 bis 43 Gew.-% des flüssigen Dispersionsmittels bestehen und daß die hochmolekulare Matrix ein Umsetzungsprodukt aus einem oder mehreren Polyisocyanaten und einer oder mehreren Polyhydroxylverbindungen und einer oder mehreren Polyhydroxylverbindungen mit einem Molekulargewicht zwischen 1 000 und 12 000 und einer OH-Zahl zwischen 20 und 112 ist, wobei das Produkt aus NCO-Funktionalität der Polyisocyanate und OH-Funktionalität der Polyhydroxylverbindungen mindestens 5,2 berträgt.

Die bevorzugten Gele sind auch dadurch gekennzeichnet, daß das flüssige Dispersionsmittel eine oder mehrere Polyhydroxylverbindungen mit einem Molekulargewicht von 1 700 bis 6 000 und einer OH-Zahl von 28 bis 84 ist, wobei die Gele zusätzlich dadurch gekennzeichnet sind, daß sie Wirkstoffe aus der Gruppe Biozide, Pharmazeutika, Naturstoffe wie etherische Öle, Duftstoffe, Farben, Detergentien und Waschhilfsmittel, Stempel- und durckfarben, Alterungsschutzmittel, Gleitmittel und Antistatika, Reinigungs- und Pflegemittel, Antifoulingmittel und Holzschutzmittel, sowie Pflanzennährstoffe, Frischhaltemittel und Wachstumsregulatoren enthalten.

Die erfindungsgemäßen Gele könne, wie schon erwähnt, in überraschend einfacher Weise durch direkte Umsetzung von Polyisocyanaten mit den genannten höhermolekularen Polyhydroxylverbindungen, gegebenenfalls in Gegenwart der Wirkstoffe, in einem Isocyanatkennzahlbereich von ca. 15 bis 60, vorzugsweise 20 bis 45, besonders bevorzugt 25 bis 40, hergestellt werden, sofern die polyurethanbildenden Komponenten (Polyisocyanate und Polyhydroxylverbindungen) zusammen polyfunktionell sind, d. h. sofern das Produkt aus Isocyanat-Funktionalität und (wie unten auf Seite 9 beschrieben zu berechnende) Polyol-Funktionalität größer als 5,2, vorzugsweise ≥ 6,2, besonders bevorzugt ≥ 8 ist, d. h. daß z. B. eine oder mehrere, mehr als bifunktionelle Komponenten in die Polyurethanbildungsreaktion eingesetzt werden. Andernfalls entstehen keine Gele aus kovalent vernetzter Polyurethanmatrix und nicht umgesetzten Polyolen, sondern die aus der Polyurehtanchemie an sich bekannten, flüssigen OH-Präpolymere.

Im allgemeinen müssen die polyurethanbildenden Komponenten umso höherfunktionell sein, je niedriger die Isocyanatkennzahl liegt, wobei das eingesetzte Polyol primäre und/oder sekundäre OH-Gruppen aufweisen kann. Im Falle der Verwendung von Gemischen von Polyolen mit primären und sekundären OH-Gruppen ist zu beachten, daß die primären hydroxylverbindungen bevorzugt mit der Isocyanatkomponente reagieren, so daß unter « Funktionalität der Polyolkomponente » dann im wesentlichen die OH-Funktionalität des primären Polyols zu verstehen ist. Zur Berechnung der Isocyanatkennzahl soll im Sinne der vorliegenden Erfindung jedoch jeweils die Gesamtmenge der Polyolkomponente herangezogen werden.

Bei der Herstellung der Polyurethanmatrix soll das Produkt aus Isocyanat-Funktionalität und, wie oben beschrieben, zu berechnender Polyol-Funktionalität mindestens 5,2, vorzugsweise mindestens 6,2, insbesondere mindestens 8, besonders bevorzugt sogar mindestens 10, betragen.

Ein Funktionalitätsprodukt von 5,2 wird beispielsweise bei dem erfindungsgemäß obersten Kennzahlbereich von ca. 60 erreicht, wenn man eine Polyolkomponente mit der Funktionalität von 2,6 und ein Diisocyanat einsetzt.

Im Falle einer Isocyanatkennzahl von 50 und rein primärer und sekundärer Polyolkomponente sollte das Produkt der Funktionalitäten mindestens 6,2, vorzugsweise 8 betragen ; im Falle einer Isocyanatkennzahl von 30 und rein primärer oder sekundärer Polyolkomponente, mindestens 9, vorzugsweise mindestens 10. Näheres ist in dieser Hinsicht den Ausführungsbeispielen zu entnehmen.

Gegenstand der Erfindung ist auch ein Verfahren zur Herstellung von gegebenenfalls wirkstoffhaltigen, im wesentlichen wasserfreien Gelmassen, dadurch gekennzeichnet, daß man

a) ein oder mehrere Di- und/oder Polyisocyanate mit

b) einer oder mehreren Polyhydroxylverbindungen mit einem Molekulargewicht zwischen 1 000 und 12 000, und einer OH-Zahl zwischen 20 und 112,

c) gegebenenfalls 0,1 bis 50 Gew.-% an Wirkstoffen,

d) gegebenenfalls Katalysatoren für die Reaktion zwischen Isocyanat- und Hydroxylgruppen,

e) sowie gegebenenfalls aus der Polyurethanchemie an sich bekannten Füll- und Zusatzstoffen

umsetzt, wobei die Isocyanatkennzahl zwischen 15 und 60 liegt, das Produkt der Funktionalitäten der Polyurethan-bildenden Komponenten mindestens 5,2 beträgt und die Polyhydroxylverbindungen im wesentlichen frei an Hydroxylverbindungen mit einem Molekulargewicht unter 800 sind.

Ein bevorzugtes Verfahren ist dadurch gekennzeichnet, daß die Polyhydroxylverbindungen ein Molekulargewicht zwischen 1 700 und 6 000 aufweisen, das Produkt der Funktionalitäten der Polyurethan-bildenden Komponenten mindestens 6,2 berträgt und daß gegebenenfalls 0,5 bis 35 Gew.-% an Wirkstoffen in Polyolverbindungen gelöst oder dispergiert verwendet werden.

Dieses Verfahren ist dadurch gekennzeichnet, daß Wirkstoffe aus der Gruppe der Biozide, Pharmazeutika, Naturstoffe wie etherische Öle, Duftstoffe, Farben, Detergentien und Waschhilfsmittel, Stempel- und Druckfarben, Alterungsschutzmittel, Gleitmittel und Antistatika, Reinigungs- und Pflegemittel, Antifoulingmittel und Holzschutzmittel, sowie Pflanzennährstoffe, Frischhaltemittel und Wachstumsregulatoren verwendet werden, welche frei von reaktiven Gruppen sind, die unter den Bedingungen der Gelbildung weitgehend oder vollständig unter Fixierung reagieren.

Die Konsistenz der Erfindungsgemäßen Gele kann zwischen einem gelee- oder gallertartigen und einem mehr oder minder hochelastischen Zustand liegen. Dieser breite Bereich wird, wie in den Ausführungsbeispielen erläutert ist, bei Variation der Isocyanatkennzahl und der Funktionalität der Ausgangskomponenten überstrichen.

Es ist besonders überraschend, daß die erfindungsgemäßen Gele außerordentlich stabil sind. Auch nach längerer Lagerung tritt keine wesentliche Phasentrennung ein. Das Dispersionsmittel Polyol ist also sehr fest im Gel festgehalten. Durch geeignete Auswahl der Mischungspartner können Gele erhalten werden, bei denen eine Abgabe des Dispersionsmittels auch bei Temperaturen von 50 bis 100 °C nicht erfolgt. Infolge der Unlöslichkeit in Dimethylformamid kann man davon ausgehen, daß die Polymerketten in den erfindungsgemäßen Gelen mindestens teilweise kovalent vernetzt sind, während der restliche Teil der Polymerketten bzw. der freien Polyole über Nebenvalenzkräfte oder mechanische Verschlaufungen gebunden sind. Es erscheint sehr überraschend, daß offenbar ein Großteil der Polyole auch nach der Reaktion mit Polyisocyanaten noch ohne Polyurethanbildung in der Polyurethanmatrix vorliegt, da er als solcher z. B. extrahiert werden kann.

Das bzw. die Polyole erfüllen, wie erläutert, neben ihrer Funktion als Aufbaukomponente für die Polyurethanmatrix zusätzlich gegebenenfalls noch die Rolle des Dispersionsmittels, das auch für die Löslichkeit, Migration und Abgabe der Wirkstoffe aus dem Gel eine wesentliche Rolle spielt.

Bei den erfindungsgemäß zu verwendenden höhermolekularen Polyolen handelt es sich vorzugsweise um die in der Polyurethanchemie an sich bekannten, bei raumtemperatur oder wenig oberhalb flüssigen Polyhydroxypolyester, -polyether, -polythioether, -polyacetale, -polycarbonate oder -polyesteramide des oben angegebenen Molekulargewichtsbereichs, OH-Zahlbereichs und OH-Funktionalität.

Die in Frage kommenden Hydroxylgruppen aufweisenden Polyester sind z. B. Umsetzungsprodukte von mehrwertigen, vorzugsweise zweiwertigen und gegebenenfalls zusätzlich drei- und vierwertigen Alkoholen mit mehrwertigen, vorzugsweise zweiwertigen Carbonsäuren. Anstelle der freien Polycarbonsäuren können auch die entsprechenden Polycarbonsäureanhydride oder entsprechende Polycarbonsäureester von niedrigen Alkoholen oder deren Gemische zur Herstellung der Polyester verwendet werden. Die Polycarbonsäuren können aliphatischer, cycloaliphatischer, aromatischer und/oder heterocyclischer Natur sein und gegebenenfalls, z. B. durch Halogenatome, substituiert und/oder ungesättigt sein.

4

Als Beispiele für solche Polycarbonsäuren und deren Derivate seien genannt: Adipinsäure, Sebacinsäure, Phthalsäure, Phthalsäureanhydrid, Tetrahydro- oder Hexahydrophthalsäureanhydrid, Isophthalsäure, Trimellitsäure, Maleinsäureanhydrid, di- und trimerisierte ungesättigte Fettsäuren, Terephthalsäuredimethylester und Terephthalsäure-bis-glykolester.

Als mehrwertige Alkohole kommen z. B. Ethylenglykol, Propylenglykol, Butandiol-1,4 und/oder -2,3, Hexandiol-1,6, Neopentylglykol, 1,4-Bis-Hydroxymethylcyclohexan, 2-Methyl-1,3-propandiol, Glycerin, Trimethylolpropan, Hexantriol-1,2,6, Pentaerythrit, Chinit, Mannit, und Sorbit, Formit, Methylglykosit, ferner Di-, Tri-, Tetra- und höhere Poly-ethylen-, Poly-propylen, sowie Poly-butylen-Glykole in Frage.

Die Polyester können anteilig endständige Carboxylgruppen aufweisen. Auch Polyester aus Lactonen, z. B. ε-Ca-prolacton oder Hydroxycarbonsäuren, z. B. ε-Hydroxycapronsäure, sind einsetzbar.

Auch die erfindungsgemäß in Frage kommenden, mindestens 2, in der Regel 2 bis 8, vorzugsweise 2 bis 3, Hydroxylgruppen aufweisenden Polyether sind solche der an sich bekannten Art und werden z. B. durch Polymerisation von Epoxiden wie Ethylenoxid, Propylenoxid, Butylenoxid, Tetrahydrofuran, Styroloxid oder Epichlorhydrin mit sich selbst, z. B. in Gegenwart von Lewis-Katalysatoren, oder durch Anlagerung dieser Epoxide, vorzugsweise ethylenoxid und Propylenoxid, gegebenenfalls im Gemisch oder nacheinander, an Startkomponenten mit reaktionsfähigen Wasserstoffatomen wie Wasser, Alkohole, Ammoniak oder Amine, z. B. ethylenglykol, Propylenglykol, Diethylenglykol, Dimethylolpropan, Glycerin, Sorbit, Succrose, Formit oder Formose, sowie 4,4'-Dihydroxy-diphenylpropan, Anilin, Ethylendiamin oder Ethanolamin hergestellt. Auch OH-Gruppen aufweisende Polythioether, Polybutadiene, Polyacetale, Polycarbonate oder Polyesteramide sind einsetzbare Ausgangsprodukte. Auch bereits Urethan- und/oder Harnstoffgruppen enthaltende Polyhydroxylverbindungen, sowie gegebenenfalls modifizierte natürliche Polyole, wie Ricinusöl, sind geeignet.

Erfindungsgemäß können gegebenenfalls auch Polyhydroxylverbindungen eingesetzt werden, in welchen hochmolekulare Polyaddukte bzw. Polykondensate oder Polymerisate in feindisperser oder gelöster Form enthalten sind. Derartige Polyhydroxylverbindungen werden z. B. erhalten, wenn man Polyadditionsreaktionen (z. B. Umsetzungen zwischen Polyisocyanaten und aminofunktionellen Verbindungen) bzw. Polykondensationsreaktionen (z. B. zwischen Formaldehyd und Phenolen und/oder Aminen) in situ in den obengenannten, Hydroxylgruppen aufweisenden Verbindungen ablaufen läßt.

Auch die durch Vinylpolymerisate modifizierten Polyhydroxylverbindungen, wie sie z. B. die Polymerisation von Styrol und/oder Acrylnitril in Gegenwart von Polyethern oder Polycarbonatpolyolen erhalten werden, sind für das erfindungsgemäße Verfahren geeignet.

Vertreter der genannten erfindungsgemäß zu verwendenden höhermolekularen Polyhydroxylverbindungen sind z. B. in High Polymers, Vol. XVI, « Polyurethanes, Chemistry and Technology », verfaßt von Saunders — Frisch, Interscience Publishers, New York, London, Band I, 1962, Seiten 32-42 und Seiten 44-54 und Band II, 1964, Seiten 5-6 und 198-199, ferner im Kunststoff-Handbuch Band VII, Vieweg-Höchtlen, Carl-Hanser-Verlag, München, 1966, z. B. auf den Seiten 45 bis 71, sowie in der DE-OS 29 20 501, Seite 17 bis 24 aufgeführt. Selbstverständlich können Mischungen der obengenannten Verbindungen, z. B. Mischungen von Polyethern und Polyestern, eingesetzt werden.

Bevorzugt werden erfindungsgemäß die in der Polyurethanchemie an sich bekannten Polyhydroxypolyether der genannten Art mit 2 bis 6, besonders bevorzugt mit etwa 2 bis 3 Hydroxylgruppen vom Molekül und einem statistisch oder segmentiert eingebauten Ethylenoxidgehalt von mindestens 10 Gew.-%, vorzugsweise mehr als 15, besonders bevorzugt mit mindestens 20 Gew.-%, als höhermolekulare Polyole eingesetzt. Ganz besonders bevorzugt werden Polypropylenetherpolyole mit mindestens 20 Gew.-% Ethylenoxid, bei denen mindestens 15 Gew.-% der OH-Endgruppen primäre Hydroxylgruppen sind.

Der Gehalt an Polyolen in der erfindungsgemäß zu verwendenden, gelbildenden Mischung beträgt etwa 80-99 Gew.-%, vorzugsweise etwa 85 bis 98 Gew.-%, bezogen auf das Gesamtgewicht der gelbildenden Mischung aus Polyurethanausgangskomponenten.

Bei den in den erfindungsgemäßen Gelen zu verwendenden organischen Di- und/oder Polyisocyanaten handelt es sich um die in der Polyurethan-Chemie an sich bekannten aliphatischen, cycloaliphatischen, araliphatischen, aromatischen und heterocyclischen Di- bzw. Polyisocyanate, wie sie z. B. von W. Siefken in Justus Liebigs Annalen der Chemie, 562, Seiten 75 bis 136 beschrieben werden, wobei die Diisocyanate als Monomere oder in modifizierter Art, z. B. biuretisiert, allophanatisiert, carbodiimidisiert, trimerisiert oder polyolmodifiziert, Verwendung finden können.

Beispielhaft seien genannt: 1,6-Hexamethylendiisocyanat, 1,12-Dodecandiisocyanat, ferner Cyclobutan-1,3-diisocyanat, Cyclohexan-1,3- und -1,4-diisocyanat, sowie beliebige Gemische dieser Stellungs- und/oder Stereoisomeren, 1-Isocyanato-3,3,5-tri-methyl-5-isocyanatomethyl-cyclohexan, 2,4- und/oder 2,6-Hexahydrotoluylendiisocyanat, Hexahydro-1,3- und/oder- 1,4-phenylen-diisocyanat, Perhydro-2,4'- und/oder- 4,4'-diphenylmethan-diisocyanat, sowie beliebige Gemische dieser Stellungs- und/oder Stereoisomeren, ferner 1,3- und 1,4-Phenylendiisocyanat, 2,4- und 2,6-Toluylendiisocyanat, Diphenylmethan-2,4'- und/oder -4,4'-diisocyanat, sowie beliebige Gemische ihrer Isomeren, und Naphthylen-1,5-diisocyanat.

Ferner kommen beispielsweise in Frage: Triphenylmethan-4,4',4"-triisocyanat, Polyphenyl-polymethylenpolyisocyanate, wie sie durch Anilin-Formaldehyd-Kondensation und anschließende Phosgenierung erhalten werden, m- und p-Isocyanatophenylsulfonyl-isocyanate, perchlorierte Arylpolyisocyanate, Carbodiimidgruppen aufweisende Polysocyanate, Norbornandiisocyanate, Allophanatgruppen aufweisende Polyisocyanate, Isocyanuratgruppen aufweisende Polyisocyanate, urethangruppen aufweisende Polyisocyanate, acylierte Harnstoffgruppen aufweisende Polyisocyanate, Biuretgruppen aufweisen Polyisocyanate, durch Telomerisationsreaktionen hergestellte Polyisocyanate, Estergruppen aufweisende Polyisocyanate, Umsetzungsprodukte der o.g. Isocyanate mit Acetalen und polymere Fettsäureester enthaltende Polyisocyanate in Betracht. Diese für die umsetzung geeigneten Polyisocyanate werden eingehend in der DE-OS 29 20 501, Seite 13, Zeilen 13 bis Seite 16, Zeile 2 beschrieben. Bevorzugte aromatische Di- und Triisocyanate sind 2,4- und/oder 2,6-Toluylendiisocyanat, und 4,4'- und/oder 2,4'-Diphenylmethandiisocyanat und ihre modifizierten Typen, sowie ihre mit tri- und tetrafunktionellen Polyolen hergestellten mehrfunktionellen Derivate oder Trimerisierungsprodukte.

Bevorzugte Polyisocyanate sind z. B. 1,6-Hexamethylendiisocyanat, Isophorondiisocyanat, Methylcyclohexan-2,4- und/oder -2,6-diisocyanat, Dicyclohexylmethan-2,4'- und/oder -4,4'-diisocyanate und ihre biuretisierten oder trimerisierten polyfunktionellen Derivate.

Alle obengenannten Di- und/oder Polyisocyanate können auch in beliebigen Gemischen eingesetzt werden.

Der Gehalt an Di- und/oder Polyisocyanaten in den gelbildenden Mischungen aus Polyolen und Polyisocyanaten beträgt ca. 1 bis 20 Gew.-%, vorzugsweise 2 bis 15 Gew.-%, bezogen auf das Gesamtgewicht der Mischung.

Bei den zur Gelbildung zu verwendenden Katalysatoren für die Reaktion zwischen Hydroxyl- und Isocyanatgruppen handelt es sich vorzugsweise um solche der in der Polyurethanchemie an sich bekannten Art, z. B. tertiäre Amine, wie Triethylamin, N-Tetramethyl-ethylendiamin, 1,4-Diaza-bicyclo-(2,2,2)-octan, N,N-Dimethylbenzylamin, N-Methyl-N'-dimethylaminoethylpiperazin, Pentamethyldiethylentriamin, oder auch als Katalysatoren bekannte Mannichbasen aus sekundären Aminen, wie Dimethylamin und Aldehyden (Formaldehyd) oder Ketonen (Aceton) und Phenolen in Frage, ferner Silaamine mit Kohlenstoff-Silicium-Bindungen, z. B. 2,2,4-Trimethyl-2-silamorpholin und 1,3-Diethyl-amino-methyltetramethyl-disiloxan. Erfindungsgemäß kommen auch organische Metallverbindungen, insbesondere organische Zinnverbindungen, als Katalysatoren verwendet werden, z. B. Zinn-(II)-acetat, Zinn-(II)-ethylhexoat und die Zinn-(IV)-Verbindungen, z. B. Dibutylzinndichlorid, Dibutylzinndilaurat, Dibutylzinnmaleat in Betracht. Weiter geeignete Katalysatoren sind in der DE-OS 29 20 501 auf den Seiten 29, Zeile 5 bis Seite 31, Zeile 25 beschrieben.

Die Katalysatoren werden vorzugsweise in einer Menge zwischen 0,05 und 10 Gew.-%, bezogen auf das Gesamtgewicht des Gels, eingesetzt. Selbstverständlich können alle Katalysatoren als Gemische eingesetzt werden.

Als erfindungsgemäß gegebenenfalls als Zusatzstoffe einzusetzende Wirkstoffe können beispielsweise folgende Substanzgruppenb bzw. Substanzen eingesetzt werden :

1. Biozide, wie z. B. Bakterizide, Fungizide, Algizide, Herbizide, Viruszide, Larvizide, Nematizide, Ektoparasitizide wie Tickizide oder Insektizide.

2. Pharmazeutika und/oder hautpflegende und -schützende Mittel, wie z. B. Antimykotika, Antiallergika, Antirheumatika, Antiseptika, Lokalanästhetika, durchblutungsfördernde Mittel, Venenmittel, Wundbehandlungsmittel, Juckreiz-stillende Mittel und Dermatika ; weiterhin Feuchtigkeit enthaltende Agentien, UV-Strahlen absorbierende Substanzen, Bakteriostatika, Kosmetika sowie desodorisierende Substanzen wie Halogenphenole oder salicylsäurederivate oder desinfizierende Substanzen.

3. Naturwirkstoffe, wie etherische Öle, z. B. Eukalyptusöl, Mentholöle, Lockstoffe (Pheromone), Vitamine oder Enzyme.

4. Duftstoffe natürlicher oder synthetischer Art, worunter etherische Öle, Parfums oder Riechstoffe aus bekannten duftenden Einzelkomponenten oder Kompositionen zu verstehen sind, z. B. Anisöl, Bergamotteöl, Kampferöl, Nelkenöl, Lemongrasöl, Lavendelöl, Pfefferminzöl, Rosenöl oder Zimtöl ; weitere geeignete Komponenten sind in der DE-OS 25 21 265 angeführt.

5. Stempel- und Blockfarben, bzw. Tinte und Malstifte auslöschende Substanzen.

6. Reinigungs- und Pflegemittel für Leder und Kunststoffe, z. B. gegebenenfalls gefärbte Wachse, Fleckentfernungsmittel.

7. Alterungsschutzmittel, z. B. Antioxidantien wie Dodecylgallat oder tert.-Butyl-substituierte Phenole, UV-Absorber, Lichtschutzmittel, Antistatika wie ethoxylierte Alkylphenole und Konservierungstoffe.

8. Pflanzennährstoffe wie anorganische Salzgemische, ferner Frischhaltemittel für Blumen und Wachstumsregulatoren.

9. Antifoulingmittel und Holzschutzmittel : z. B. Pulver von Kupfer-, Quecksilber- oder Zinn-Verbindungen, sowie Pentachlorphenole und Dinitrophenole.

10. Detergentien und Waschhilfsmittel, wie Alkylarylsulfonate, Fettalkoholsulfate, Fettalkohol-Ethylenoxid-Addukte, Weichspülmittel, Formspülmittel, Schaumdämpfer und Aufheller.

11. Fotohärtbare Gemische.

Der Gehalt an Wirkstoffen in den erfindungsgemäßen Gelmassen beträgt 0,1 bis 50 Gew.-%, vorzugsweise 0,5 bis 35 Gew.-% und besonders 0,75 bis 25 Gew.-%, bezogen auf das Gesamtgewicht der Gelmasse (Komponenten 1-4). Es kann jedoch bei sehr aktiven Zusatzstoffen, z. B. Pheromonen, auch niedriger (z. B. < 0,01 %) sein.

Eine mögliche Begrenzung der Art der einzusetzenden Wirkstoffe ergibt sich bei solchen Wirkstoffen, welche so reaktive chemische Gruppen enthalten, daß sie unter den Bedingungen der gelbildenden Polyurethanreaktion weitgehend oder vollständig fixiert werden und bleiben und die Wirkstoffe nicht mehr zu entweichen in der Lage sind.

Als in den erfindungsgemäßen Gelen gegebenenfalls zusätzlich enthaltene Füll- und Zusatzstoffe sind die in der Polyurethanchemie an sich bekannten Stoffe zu verstehen, wie z. B. Füllstoffe, Pigmente und Kurzfasern auf anorganischer und organischer Basis, Metallpulver, färbende Agentien wie Farbstoffe und Farbpigmente, wasserbindende Mittel, oberflächenaktive Substanzen wie Silikone, ferner Flammschutzmittel oder flüssige Streckmittel mit einem Siedepunkt über 150 °C. Als organische Füllstoffe seien beispielsweise Schwerspat, Kreide, Gips, Soda, Titandioxid, Zeolithe, Quarzsand, Kaolin, Ruß und Mikroglaskugeln genannt. Von den organischen Füllstoffen können z. B. Pulver auf Basis von Polystyrol, Polyvinylchlorid, Harnstoff-Formaldehyd und Polyhydrazodicarbonamid eingesetzt werden. Als Kurzfasern kommen z. B. Glasfasern von 0,01 bis 1 mm Länge oder Fasern organischer Herkunft, z. B. Polyester-, Polyamid-, Aramid- oder Kohlenstoff-Fasern in Frage. Metallpulver, wie z. B. Eisen- oder Kupferpulver, können ebenfalls mitverwendet werden. Zur Einfärbung der Gele können nichtmigrierende Farbstoffe und Farbpigmente auf organischer oder anorganischer Basis verwendet werden. Als oberflächenaktive Substanzen seien z. B. Zellulosepulver, Aktivkohle und Kieselsäurepräparate genannt. Als Flammschutzmittel können z. B. Natriumpolymethaphosphate zugesetzt werden. Als flüssige Streckmittel bzw. Weichmacher können die üblichen Verbindungen mitverwendet werden, z. B. Alkyl-, Alkoxy- oder Halogen-substituierte aromatische Verbindungen, wie Dodecylbenzol, ortho-Dichlorbenzol, chloriertes Paraffin oder Dodecylsulfonsäureester. Weiterhin können als flüssige Streckmittel auch höhermolekulare Polyole eingesetzt werden, deren Hydroxylgruppen verethert, verestert oder urethanisiert sind. Der Gehalt an diesen Steck- und Füllstoffen beträgt bis zu 50 %, vorzugsweise unter 25 %, bezogen auf die Summe aus 1 + 2.

Für die Formulierungen der dem jeweiligen Anwendungszweck angepaßten erfindungsgemäßen Gelmassen können weiterhin die unterschiedlichsten Hilfsmittel mitverwendet werden. Sollen z. B. Pharmazeutika den erfindungsgemäßen Gelen inkorporiert werden, so können Resorptionshilfsmittel wie Phosphorlipide, Löslichkeitsverbesserer wie Polyethylenglykole oder Polypropylenglykole, Emulgatoren wie Glycerinfettsäureester, Spreitmittel wie Silikonöle, Fettsäureester oder Triglyceride, sowie hautpflegende Substanzen wie 2-Octyl-dodecanol bei der Gelbildung mit zugesetzt werden.

Bei Biocid-haltigen Formulierungen, die feste Wirkstoffe enthalten sollen, ist es vorteilhaft, gegebenenfalls Spreitmittel und insbesondere Weichmacher, wie Dibutylphthalat, bei der Gelbildung zuzusetzen.

Als Spreitmittel kommen z. B. folgende Substanzen in Beracht : Silikonöle verschiedener Viskosität, Fettsäureester wie Laurinsäurehexylester, Dipropylenglykolpelargonat, Ester verzweigter Fettsäuren mittlerer Kettenlänge mit gesättigten $C_{16}$-$C_{18}$-Fettalkoholen, wie Isopropylmyristat, Isopropylpalmitat, Capryl-/Caprinsäureester von gesättigten Fettalkoholen der Kettenlänge $C_{12}$ bis $C_{18}$, Isopropylstearat, Ölsäuredecylester, wachsartige Fettsäureester wie Adipinsäurediisopropylester, Triglyceride, wie Capryl-/Caprinsäuretriglycerid, Triglyceridgemische mit Pflanzenfettsäuren der Kettenlänge $C_8$ bis $C_{12}$ oder anderen speziell ausgewählten natürlichen Fettsäuren, Partialglyceridgemische oder Monoglyceride, ferner Fettalkohole, wie Isotridecyl-Alkohol, 2-Octyl-dodecanol oder Oleylalkohol, oder Fettsäuren wie Ölsäure oder Stearinsäure. Besonders gut spreitende Öle sind Isopropylmyristat, Isopropylstearat, Isopropylpalmitat, Laurinsäurehexylester, Ölsäuredecylester, Dibutylstearat, Dibutylsebacat, Paraffinöl, Ethylhexyl-palmitat/-stearat, bzw. Iso-tridecyl-stearat.

Die Gesamtmenge an Füll- und Zusatzstoffe, einschließlich der Wirkstoffe soll 100 Gew.-%, bezogen auf die Summe PU-Matrix und Dispersionsmittel, nicht überschreiten.

Die Herstellung der erfindungsgemäßen, wirkstoffhaltigen Gelmassen kann kontinuierlich oder diskontinuierlich vorgenommen werden. Die Arbeitsweise hängt u. a. von der Form ab, die man den erfindungsgemäßen Gelen im Hinblick auf ihre Anwendung geben möchte.

Man kann nach dem One-shot- oder dem prepolymer-Verfahren arbeiten. Beim One-shot-Verfahren werden alle Komponenten, d. h. Polyole, Di- und/oder Polyisocyanate, Wirkstoffe, Katalysatoren und gegebenenfalls weitere Füll- und Zusatzstoffe auf einmal zusammengegeben und intensiv miteinander vermischt, wobei die Wirkstoffe vorzugsweise in den Polyolkomponenten gelöst oder dispergiert werden.

Beim Prepolymerverfahren sind zwei Arbeitsweisen möglich. Entweder stellt man zunächst ein Isocyanat-Prepolymer her, indem man einen entsprechenden Anteil der Polyolmenge (+ Wirkstoff) der gesamten, für die Gelbildung vorgesehenen Isocyanatmenge umsetzt, und fügt dann dem erhaltenen Prepolymer die restliche Menge an Polyol (gegebenenfalls weitere Wirkstoffe), sowie gegebenenfalls weitere Füll- und Zusatzstoffe zu und mischt intensiv, oder man setzt die gesamte, für die Gelbildung vorgesehene Menge an Polyol (+ Wirkstoff) mit einem Teil der Polyisocyanatmenge zu einem OH-Prepolymer um und mischt anschließend die restliche Menge an Polyisocyanat zu.

Eine erfindungsgemäß besonders vorteilhafte Arbeitsweise ist eine Variante aus dem One-shot-Verfahren und dem OH-Prepolymer-Verfahren. Hierbei werden das Polyol bzw. Polyolgemisch, die Wirkstoffe, gegebenenfalls die Füll- und Zusatzstoffe, der Katalysator und zwei verschiedene Diisocyanate in einem Schuß zusammengegeben und intensiv vermischt, wobei ein Di- oder Polyisocyanat aromatischer und ein Di- und/oder Polyisocyanat aliphatischer Natur ist. Man kann davon ausgehen, daß durch die stark unterschiedliche Reaktivität der beiden Polyisocyanate zunächst ein Hydroxyl-Prepolymer

7

entsteht, das sodann innerhalb von Minuten mit dem anderen Polyisocyanat unter Gelbildung reagiert. Es werden hierdurch Gele mit besonders hoher Zähigkeit erhalten.

Bei diesen Verfahrensweisen kann die Förderung, Dosierung und Mischung der Einzelkomponenten oder Komponentengemische mit den für den Fachmann in der Polyurethan-Chemie an sich bekannten Vorrichtungen erfolgen.

Für den Fachmann besonders überraschend ist es, daß auch bei relativ niedrigen Isocyanatkennzahlen (z. B. 30) und einer Polyolkomponente mit einheitlich reaktiven OH-Gruppen (so daß keine selektive Reaktion eines Teils der Polyolkomponente mit dem Polyisocyanat zu erwarten ist) Gele mit einer hochmolekularen, vernetzten, in Dimethylformamid unlöslichen Matrix und nicht bloß durch Urethangruppen modifizierte flüssige Polyole (OH-Prepolymere) erhalten werden.

Es ist dabei zur Erzielung einer guten Matrixstruktur vorteilhaft, die Umsetzung zwischen den Polyolen und den Polyisocyanaten bei relativ niedrigen Temperaturen, z. B. unter 50 °C, vorzugsweise bei Raumtemperatur, durchzuführen.

Will man für die Anwendung z. B. Formteile herstellen, so ist die diskontinuierliche Arbeitsweise anzuraten. Soll das erfindungsgemäße Polyurethangel jedoch in Stücken geeigneter Abmessungen hergestellt werden, dann ist eine kontinuierliche Verfahrensweise oft günstiger. In diesem Fall produziert man zunächst eine endlose Folie oder Platte, die man anschließend in einzelne Stücke zerteilen kann.

Bei der kontinuierlichen Herstellung kann das gegebenenfalls Wirkstoff enthaltende gelfähige Gemisch auch, bevor es durch die Gelbildung erstarrt, gesprüht, gegossen oder gerakelt werden. Hierbei kann das gelfähige, wirkstoffhaltige Gemisch auf die verschiedenartigsten Materialien auf Basis von natürlichen oder synthetischen Rohstoffen aufgebracht werden, z. B. auf Matten, Vliese, Gewirke, Gestricke, Schaumfolien, Kunststoff-Folien bzw. -platten, oder in gewünschte Formen eingegossen werden.

Die Bedingungen während der Gelbildung lassen sich auch in der Weise variieren, daß man entweder kompakte oder geschäumte Gele erhält. Wird z. B. Luft in das gelfähige Gemisch eingeschlagen, so erhält man Schaumgele.

Man kann die Erfindung auch zum Abformen von Gegenständen durch Umgießen des abzuformenden Körpers mit einer gelbildenden Masse und Entnahme des Formkörpers nach der Gelbindung, benutzen. Diese Verwendung ist dadurch gekennzeichnet, daß man den Körper mit einer Mischung aus

a) einem oder mehreren Di- und/oder Polyisocyanaten,

b) einer oder mehreren Polyhydroxylverbindungen mit einem Molekulargewicht zwischen 1 000 und 12 000, vorzugsweise zwischen 1 700 und 6 000, und einer OH-Zahl zwischen 20 und 112, vorzugsweise zwischen 28 und 84, besonders bevorzugt zwischen 30 und 56,

c) gegebenenfalls Katalysatoren für die Reaktion zwischen Isocyanat- und Hydroxylgruppen sowie gegebenenfalls,

d) aus der Polyurethanchemie an sich bekannten Füll- und Zusatzstoffen

gegebenenfalls in mehrere Schichten mit gegebenenfalls unterschiedlicher Zusammensetzung umgießt, wobei diese Mischung im wesentlichen frei ist an Hydroxylverbindungen mit einem Molekulargewicht unter 1 000, die Isocyanatkennzahl zwischen 15 und 60 liegt und das Produkt der Funktionalitäten der polyurethanbildenden der Funktionalitäten der polyurethanbildenden Komponenten mindestens 5,2, vorzugsweise 6,2, insbesondere 8, besonders bevorzugt 10, beträgt.

Die Anwendung der Gele geschieht nach den in der Abform- bzw. Doubliertechnik üblichen Methoden. Hierbei kann das gelfähige Gemisch, bevor es durch die Gelbildung erstarrt, gegossen oder auch gesprüht werden. Das Gel kann auch durch die verschiedenartigsten Materialien auf Basis natürlicher oder synthetischer Rohstoffe, wie z. B. Vliese, Gewirke, Gestricke, Gewebe, Schaumfolien, Platten oder Matten, verstärkt werden, wobei die Materialien im Inneren des Gels oder als äußere Schicht auf das Gel angebracht werden können. Die Gelmasse kann auch in Schichten nacheinander auf das abzuformende Modell aufgebracht werden. Hierbei wird auf das Modell zur genauen Abformung zunächst eine kompakte Gelschicht aufgetragen.

Sodann kann als zweite Schicht z. B. mit Luft stark angereicherte gelfähige Masse aufgebracht werden, die zu einem Schaumgel führt und dadurch das Gewicht der Gelformen verringert. Als zweite Schicht kann andererseits auch ein füllstoffhaltiges Gel zur Verstärkung der herzustellenden Gelform aufgetragen werden.

Die erfindungsgemäßen Gele eignen sich zur genauen Abformung von Modellen aus den unterschiedlichsten Materialien, wie z. B. aus Gips, Holz, Beton, Stahl, Kunststoffen wie Epoxiden oder Polyurethanen, Stein, Keramik oder Metallen wie Kupfer und Eisen, sowie von Knochen, Gelenken, Gebiß- und Zahnformationen.

Ein wesentlicher Vorteil der erfindungsgemäßen Polyol-Gele gegenüber bekannten wasserfreien Abformassen, wie z. B. Massen auf Silikonbasis, liegt in der niedrigeren Viskosität der gelbildenden Mischung. Dadurch werden auch sehr feine Vertiefungen in der Modelloberfläche abgeformt. Ein weiterer Vorteil der neuen Gele ist, daß sie kürzere Reaktionszeit aufweisen und somit eine schnellere Entformung des abzuformenden Modells ermöglichen. Die Herstellung einer einen Hohlraum enthaltenden Form erfordert somit weniger Zeitaufwand.

Die erfindungsgemäßen Polyol-Gele unterscheiden sich durch ihre höhere Elastizität vorteilhaft von wäßrigen Gelen, wie z. B. Agar-Agar-Gel. Dadurch wird auch die Abformung von dünnen Stegen und Hinterschnitten einwandfrei ermöglicht ; ein Einreißen der Gelform beim Entfernen des abzuformenden Modells tritt nicht auf.

Ein weiterer Vorteil der erfindungsgemäßen Gele gegenüber Gelen auf Wasserbasis liegt in der Dimensionsstabilität bei offener Lagerung.

Die erfindungsgemäßen Polyolgele, können weiterhin als Eingußmassen für medizinische und biologische Präparate, wie zum Beispiel Käfer, Schmetterlinge, innere Organe und Gewebeproben, verwendet werden. Die bisher hierfür eingesetzten Kunstharze z. B. auf Basis von Epoxidharzen weisen verschiedene Nachteile auf, insbesondere zu hohe Wärmeentwicklung und zu hoher Schrumpf ; andererseits zeigen Naturstoffgele, wie Gelatine, eine unzureichende Langzeitkonsistenz, d. h., nach Monaten kann bereits ein Zerfall der Gele erfolgen.

Die erfindungsgemäßen Gele zeichnen sich für diese Anwendung insbesondere dadurch aus, daß sie klardurchsichtig und nicht vergilbend sind und über Monate und Jahre die Gelkonsistenz erhalten bleibt.

Weiterhin lassen sich die erfindungsgemäßen Gele aufgrund ihrer hohen Elastizität verwenden als stoßabsorbierende Elemente, wie z. B. für Sicherheitsstoßdämpfer bei Aufzugsanlagen oder für Autostoßstangensysteme, als druckverteilende Elemente, wie z. B. für Abpolsterungen von Prothesen gegenüber Körperteilen oder für Druckwalzen, als wasserquellbare Elemente, wie z. B. für die Abdichtung von Schachtwänden im Tiefbau oder für defekte Rohrleitungen gegen das Eindringen von Wasser sowie für Schaltersysteme von automatisch arbeitenden Wasserberieselungsanlagen, als Füllsubstanz für Brustprothesen, als Einbettungs- bzw. Umhüllungssubstanz von Lichtleitfasern für die optische Nachrichtenübertragung sowie von Flüssigkristallen für beispielsweise Anzeigeflächen, als Dämmaterial für Körperschall beispielsweise im Automobilsektor oder Maschinenbau und als Füllmaterial für Heißkompressen oder Kaltkompressen für medizinische Anwendungen.

Die Anwendung der erfindungsgemäßen, gegebenenfalls wirkstoffhaltigen Gelmassen kann in den verschiedensten Formen, wie z. B. als Granulat, Folie, Platte, Block, Stab oder Formteil, erfolgen. Die Wahl hängt vom jeweiligen Anwendungszweck und der gewünschten Abgabekonzentration der Wirkstoffe ab. Hierbei können die Wirkstoffe über Wochen und Monate aus den erfindungsgemäßen Gelen herausdiffundieren und in Abhängigkeit von ihrer Flüchtigkeit an die Gasphase und/oder bei Kontakt der erfindungsgemäßen Gelmassen mit festen oder flüssigen Materialien bzw. Substanzen (z. B. die Tierhaut oder Wasser) an die kontaktierten Materialien abgegeben werden.

Die erfindungsgemäßen wirkstoffhaltigen Gele eignen sich zur längerfristigen Abgabe der inkorporierten Wirkstoffe zu den unterschiedlichsten Anwendungszwecken, wie z. B. als dermatologische Substanzen enthaltende Pflaster zur Befestigung auf der Haut, als insektizidhaltige Bänder und Platten zur Bekämpfung von Fliegen und Ungeziefer, z. B. zur Beseitigung von Zecken und Flöhen an Tieren, als duftstoffhaltige Platten und Formteile zur Beduftung von Räumen, als desodorisierende Masse zur Übertragung auf die Haut, als Druck- oder Stempelplatten geringer Trocknungstendenz, als Schuhputzmittel zum Auftragen von Farbe und Wachsen, als insektizidhaltiger Baumring gegen Insekteneinwirkung, als Gleitmittel mit Antistatikwirkung und anderes mehr.

Ein weiterer Gegenstand der Erfindung ist somit die Verwendung von wirkstoffhaltigen Gelen als Wirkstoffträger mit Depotwirkung dadurch gekennzeichnet, daß sie zu längerfristigen Abgabe der Wirkstoffe aus den Gelformteilen in Form von duftstoffhaltigen Formteilen zur Beduftung von Räumen, in Form von insektizidhaltigen Formteilen zur Bekämpfung von Fliegen und Ungeziefer, in Form von desodorisierenden Formteilen zur Übertragung auf die Haut, oder in Form von dermatologische Wirkstoffe enthaltenden Pflastern oder in Form von Druck- und Stempelplatten geringer Trocknungstendenz eingesetzt werden.

Ein wesentlicher Vorteil der erfindungsgemäßen wirkstoffhaltigen Gele gegenüber den wasserhaltigen Wirkstoffgelen ist eine höhere Stabilität von hydrolyseanfälligen aktiven Reagentien, wie z. B. Insektiziden, Pflanzenschutzmitteln, Duftstoffen oder Pharmazeutika während der Lagerung und des Wirkungszeitraumes der Gele.

Ein weiterer wesentlicher Vorteil der neuen Gelmassen ist, daß auch inkorporierte feste bzw. schwerflüchtige Wirkstoffe herauswandern und damit über einen längeren Zeitraum wirksam sein können, wenn sie eine gewisse Löslichkeit in den Polyolen als Dispergiermittel aufweisen. In dieser Hinsicht stellen die erfindungsgemäßen Gele eine wertvolle Verbesserung gegenüber solchen massiven und geschäumten Polyurethanen dar, bei deren Herstellung die reaktiven Komponenten in Mengen, die einer Isocyanatkennzahl von 70 bis 200 entsprechen, eingesetzt werden, und bei welchen keine wesentlichen Mengen an freien Polyolen der erfindungsgemäßen Effekt bewirken können, sondern im Gegenteil die hohe Vernetzungsdichte das Auswandern von festen Wirkstoffen behindert.

## Versuchsteil

Die folgenden Beispiele erläutern die vorliegende Erfindung. Mengenangaben sind als Gewichtsprozente bzw. Gewichtsteile zu verstehen, sofern nichts anderes angegeben ist.

In den Beispielen wurden die folgenden Polyisocyanate bzw. Polyole eingesetzt :

Polyisocyanat 1
1,6-Hexamethylendiisocyanat

Polyisoc

Handels biuretisiertes 1,6-Hexamethylendiisocyanat mit einer mittleren NCO-Funktionalität von 3,6, ein Gehalt von 21 % und einem mittleren Molekulargewicht (Zahlenmittel) von ca. 700 (Desmodur ayer AG).

Polyisoc

Isomerei aus 80 % 2,4- und 20 % 2,6-Toluylendiisocyanat.

Polyisoc

Durch brisierung mit Tripropylenglykol verflüssigtes 4,4'-Diisocyanato-diphenylmethan ; mittlere NC(nalität 2,05, NCO-Gehalt 23 %.

Polyisoc

Präpolyr159 Teilen Polyisocyanat 3 und 1 200 Teilen eines Polyethers der OH-Zahl 28, hergestellt dagerung von 60 Teilen Ethylenoxid und 40 Teilen Propylenoxid an Glycerin.

Die in delen verwendeten Polyether-Polyole sind in der nachfolgenden Tabelle zusammengestellt. TMFder Tabelle für Trimethylolpropan ; PF für 1,2-Propylenglykol ; Gly für Glycerin und PE für Penta

| Pol | Propylenoxid % | Ethylenoxid % | Starter-molekül | OH-Zahl | OH-Funktio-nalität |
|-----|-----|-----|-----|-----|-----|
|     | 80  | 20  | TMP | 36 | 3 |
|     | 100 | —   | PG  | 56 | 2 |
|     | 45  | 55  | TMP | 56 | 3 |
|     | 100 | —   | TMP | 56 | 3 |
|     | 90  | 10  | TMP | 56 | 3 |
|     | 85  | 15  | TMP | 56 | 3 |
|     | 83  | 17  | TMP | 34 | 3 |
|     | 100 | —   | Sorbit | 46 | 6 |
|     | 40  | 60  | Gly | 28 | 3 |
| 1   | 100 | —   | TMP/PG (84:16) | 46 | 2,75 |
| 1   | 100 | —   | PE  | 45 | 4 |
| 1   | 50  | 50  | PG  | 56 | 2 |
| 1   | 80  | 20  | PG  | 28 | 2 |
| 1   | 82  | 18  | TMP | 35 | 3 |
| 1   | 63  | 37  | Sorbit | 30 | 6 |

Polyol 1( teilverzweigter Polyester aus Adipinsäure, Diethylenglykol und TMP. Mittleres Molekulargev. 2 000 ; mittlere OH-Funktionalität : 2,3.

Beispiel 1

80 Teile ethers (1), 15 Teile Methylbutyrat als Duftstoff, 1,2 Teile Dibutylzinndilaurat und 4 Teile Polyiso() werden innerhalb 1 Minute intensiv vermischt. Nach 15 Minuten bildet sich ein elastisches Gm eines Formkörpers wie z. B. eines Kegels, einer Platte oder einer Rosennachbildung, kaniGel zur längerfristigen Beduftung von Schränken, Räumen, Automobilen oder Müllbehälterriet werden.

Beispiel 2

10 Teile F1, 40 Teile Polyether 2 und 8 Teile eines Parfumöls (aus 60 Gew.-% Isobornylacetat und 40 GewAnlagerungsproduktes von 10 Mol Ethylenoxid an 1 Mol Nonylphenol, 50 Teile Polyether (3) Teile K-Sorbinat, 1,5 Teile Dibutylzinndilaurat und 6 Teile Polyisocyanat (2) werden innerhalb 1 Nensiv vermischt. Nach 15 Minuten bildet sich ein klares, elastisches Gel, dessen Oberfläche klebfrei ist und das als Duftstoffspender zur Luftverbesserung in Räumen benutzt werden kann. Das Duftstoffgel behält seine Struktur und Wirksamkeit über Monate bei.

### Beispiel 3

Analog zu Beispiel 2 wird ein Gel hergestellt aus 10 Teilen Polyether, 4, 50 Teilen Polyether 5, der 4 Teile des Parfumöls von Beispiel 2 enthält, 40 Teilen Polyether (6), der 0,18 Teile Natriumbenzoat als Bakterizid enthält, 1,5 Teilen Dibutylzinndilaurat, und 6 Teile Polyisocyanat (2).

### Beispiel 4

100 Teile Polyether (3), 5 Teile Triethylenglykoldimethylether, 8 Teile Duftöl 83/117 (Duftrichtung Zitrone ; Produkt der Fa. Colgate Palmolive Peet Inc., USA), 2,5 Teile Dibutylzinndilaurat und 8 Teile Polyisocyanat (2) werden intensiv vermischt und in eine offene Form bis zu einer Höhe von 3 mm gegossen. Man erhält eine 3 mm starke Gelfolie, die in 1,5 × 10 cm große Streifen zerschnitten wird. Ein derartiger Streifen kann am Innenteil des Deckels einer Verpackung befestigt werden, die 4 bis 5 kg Waschmittelpulver enthält. Auf diese Weise erzielt man eine Beduftung des Waschmittels, ohne das die Gefahr einer Zerstörung des Duftstoffes durch die Bestandteile des Waschmittels (Oxydationsmittel) besteht.

### Beispiel 5

3 500 Teile Polyether (3), der 350 Teile Pentachlorphenol enthält, 700 Teile Polyether 7, der 14 Teile K-Sorbinat enthält, 2 800 Teile Polyether 2, dem 50 Teile eines hochmolekularen Polyethylenoxids zugemischt sind, und 35 Teile Dibutyl-zinn-dilaurat werden in einem Rührkessel bei 22 °C homogen gemischt.

Die Mischung wird mittels einer Zahnradpumpe einem statischen Mischer zugeführt. Aus einem getrennten Vorratsbehälter werden diesem Mischer mittels einer weiteren Zahnradpumpe gleichzeitig 473 Teile Polyisocyanat (2) so zugeführt, daß zu jeder Zeit das Mischungsverhältnis der Komponenten gleich ist und dem Verhältnis der Gesamtmengen entspricht. Die aus dem statischen Mischer ausfließende weißlich-trübe Lösung wird in eine quadratische Umhüllung gegossen. Nachdem die Gelbildungsreaktion abgeschlossen ist, wird ein weiches, formbeständiges, unter Druck deformierbares Gel erhalten, das als desodorisierender Gelstift zur Verhinderung von Schweißgeruch durch bakterielle Zersetzung benutzt werden kann.

### Beispiel 6

75 Teile Polyether (1), 20 Teile o,o-Dimethyl-o-(2,2-dichlorvinyl)-phosphorsäureester (DDVP, Insektizid), 1,2 Teile Dibutyl-zinn-dilaurat und 3,8 Teile Polyisocyanat 2 werden innerhalb von 1 Minute intensiv vermischt. Nach ca. 10 Minuten bildet sich ein elastisches Gel, das in Form eines Streifens, der in einen perforierten Kunststoffbehälter gesteckt ist, als insektizides Gel zur längerfristigen Begasung der Atmosphäre, z. B. zur Bekämpfung von Ungeziefer oder von Kakerlaken in Küchen, benutzt werden kann.

### Beispiel 7

1 000 Teile Polyether 8, der 100 Teile des Insektizids DDVP (s. Beispiel 6) enthält, 25 Teile Polyisocyanat (3) und 30 Teile Dibutylzinndilaurat werden mit Hilfe eines Laborrührers mit einer Rührscheibe bei Raumtemperatur innerhalb von 1 Minute intensiv vermischt. Man erhält ein weiches, elastisches, formstabiles Gel, das sich unter dem Einfluß einer darauf wirkenden Kraft leicht deformieren läßt.

### Beispiel 8

100 Teile Polyether 9, 4, 0 Teile Hexachlorophen und 0,48 Teile p-Hydroxybenzoesäure-ethylester enthaltend, werden mit 5,0 Teilen Polyisocyanat 4 und 2,8 Teilen Dibutyl-zinn-dilaurat zu einem weichen, elastischen, formstabilen Gel umgesetzt. Das Gel eignet sich zum Bestreichen von Haut zur Verhinderung von bakteriellen Schweißzersetzungen.

### Beispiel 9

100 Teile Polyether (1) mit einer Temperatur von 70 °C, 30 Teilen 2-Isopropoxyphenyl-N-methylcarbamat (ein Insektizid), 60 Teile Isopropylmyristat, 5 Teile Permethrinsäure-pentafluorbenzylester (ein Insektizid), 2 Teile Dibutyl-zinn-dilaurat, 0,3 Teile Eisenoxid-Pigment und 5,5 Teile Polyisocyanat (2) werden intensiv vermischt. Das Reaktionsgemisch wird in eine offene Form, die mit Synthetikleder ausgelegt ist, bis zu einer Schichtdicke von 5 mm gegossen. Nach der Erhärtung zum Gel werden 15 mm breite Streifen geschnitten, die aus einer Lederdekorschicht und einer wirkstoffhaltigen Gelschicht bestehen. Diese Streifen werden mit einer Schnalle versehen und lassen sich dann als Halsbänder gegen Flöhe und Zecken bei Haustieren wie katzen oder Hunden verwenden.

Beispiel 10

Analog zu Beispiel 1 werden unter Variation der OH- bzw. NCO-Funktionalität der Ausgangskomponenten Gele hergestellt, wobei die Isocyanat-Kennzahl jeweils 50 betrug. Die Eigenschaften der so erhaltenen Gele sind in der nachfolgenden Tabelle zusammengestellt ; « flüssig » bedeutet, daß infolge zu niedriger Funktionalität noch keine Gelstruktur ausgebildet wurde (nicht erfindungsgemäß). Als Isocyanatkomponente werden Polyisocyanat (1), Polyisocyanat (2) bzw. Gemische daraus mit der angegebenen mittleren NCO-Funktionalität verwendet ; die Polyolkomponenten bestanden aus den Polyolen 10 bzw. 11 bzw. 1 : 1-Gemischen von 2 und 10 bzw. 10 und 11, im Polyol werden jeweils 5 Gew.-% La wendelöl als Duftstoff eingesetzt.

| Funktionalität NCO / OH | 2 | 2,3 | 2,75 | 3,75 | 4 |
|---|---|---|---|---|---|
| 2 | | | | flüssig | sehr weich * |
| 2,1 | | | | sehr weich * | weich ** |
| 2,2 | | | flüssig | weich ** | hart ** |
| 2,3 | | | sehr weich * | weich ** | |
| 2,4 | | | sehr weich * | | |
| 2,6 | | flüssig | weich ** | | |
| 2,8 | | sehr weich * | | | |
| 3,1 | | weich ** | | | |
| 3,6 | flüssig | hart ** | | | |

*) erfindungsgemäß
**) erfindungsgemäß bevorzugt
Es werden Duftträgergele erhalten.

Beispiel 11

Analog zu Beispiel 10 wurde die Abhängigkeit der Gelkonsistenz von der Funktionalität für die Isocyanat-Kennzahl 30 untersucht. Als Hydroxylkomponenten wurden die Polyole 10, 11, 8 bzw. ein 1 : 1-Gemisch aus 11 und 8 verwendet, wobei die Polyole jeweils 4 Gew.-%Lavendelöl enthalten.

| Funktionalität: NCO / OH | 2,75 | 4 | 4,8 | 6 |
|---|---|---|---|---|
| 2 | | | | flüssig |
| 2,1 | | | | sehr weich* |
| 2,15 | | | flüssig | weich** |
| 2,2 | | | sehr weich* | weich–hart** |
| 2,3 | | | sehr weich* | hart** |
| 2,4 | | flüssig | weich** | hart** |
| 2,8 | flüssig | sehr weich* | weich** | hart* |
| 3,6 | sehr weich* | weich** | | |

*) erfindungsgemäß
**) erfindungsgemäß bevorzugt
Es werden Duftträgergele erhalten, welche eine langdauernde Riechstoffabgabe zeigen.

Beispiel 12

In Analogie zu Beispiel 10 wurde die Abhängigkeit der Gelkonsistenz von Isocyanatkennzahl und NCO-Funktionalität untersucht. Als Polyolkomponente wird ein 1 : 1-Gemisch der Polyole 2 und 12 eingesetzt, welche 10 Gew.-% Lavendelöl als Duftstoff enthalten. Als Isocyanatkomponenten dienen Gemische der Polyisocyanate 1 und 2 in der angegebenen mittleren NCO-Funktionalität.

| Funktionalität: NCO/Kennzahl | | 2,6 | 2,8 | 3,0 | 3,2 |
|---|---|---|---|---|---|
| 55 52,5 | Vergleich | sehr weich flüssig | | | |
| 50 47,5 | erfindungs- gemäß | sehr weich (flüssig) | weich** sehr weich* | weich** | hart** |

*) erfindungsgemäß
**) erfindungsgemäß bevorzugt

## Beispiel 13

Abhängigkeit der Gelkonsistenz von der NCO-Funktionalität bei konstanter Isocyanatkennzahl (50) und OH-Funktionalität (3).
Versuch 1
Polyolkomponente : Polyol 6
Isocyanatkomponente : Verschiedene Gemische aus Polyisocyanaten 1 und 2.
Versuch 2 :
Polyolkomponente : Polyol 4/Polyol 6 (1 : 1), (8 Gew.-% Buttersäuremethylester als Duftstoff enthalten) ;
Isocyanatkomponente : wie Versuch 1.

| NCO-Funktionalität | Versuch 1 | Versuch 2 |
|---|---|---|
| 2 | flüssig | flüssig |
| 2,1 | flüssig | sehr weich-weich |
| 2,2 | sehr weich * | weich ** |
| 2,3 | weich ** | weich-hart ** |
| 2,4 | weich-hart ** | hart ** |
| 2,6 | hart ** | hart * |
| 2,8 | hart ** | sehr hart * |

*), **) : Bedeutung sh. Beispiel 12

## Beispiel 14

Abhängigkeit der Gelkonsistenz vom Mischungsverhältnis Polyether mit primären Hydroxylgruppen/Polyether mit sekundären Hydroxylgruppen. Die Polyole enthielten 10 Gew.-% Methylbutyrat.
Isocyanatkennzahl : 35
Isocyanatkomponente : Polyisocyanat 2
Die Gele wurden analog zu Beispiel 1 hergestellt.

| Versuch | Polyol 6 (%) | Polyol 4 (%) | Gelkonsistenz *) |
|---|---|---|---|
| 1 | 0 | 100 | sehr weich |
| 2 | 5 | 95 | weich |
| 3 | 15 | 85 | weich bis hart |
| 4 | 25 | 75 | hart |
| 5 | 35 | 65 | sehr hart |
| 6 | 45 | 55 | hart |
| 7 | 75 | 25 | hart |
| 8 | 100 | 0 | weich bis hart |

## Beispiel 15

Für Versuch von Beispiel 14 wurde untersucht, wieviel des (praktisch nicht mitreagierenden) Polyols 4 bei sonst gleicher Rezeptur (10 Gew.-% Methylbutyrat im Polyolgemisch) dem Reaktionsansatz zugesetzt werden kann, so daß noch ein Gel erhalten wird. Wie die nachfolgende Tabelle zeigt, liegt die Grenze der Gelbildung für die gewählten Ausgangskomponenten bei einer Zusammensetzung, die (theoretisch berechnet) 28 Gew.-% Polyurethanmatrix und 72 Gew.-% freiem Polyol entspricht. Extraktionsversuche des Polyols zeigen praktisch Ergebnisse wie theoretisch berechnet.

| Rezeptur (Teile) | | | | | |
|---|---|---|---|---|---|
| Polyol 6 | 35 | 35 | 35 | 35 | 35 |
| Polyol 4 | 65 | 100 | 105 | 120 | 150 |
| Polyisocyanat 2 | 7 | 7 | 7 | 7 | 7 |
| Dibutylzinndilaurat | 3 | 3 | 3 | 4 | 5 |
| % Polyurethanmatrix | 38 | 29 | 28 | 25 | 21 |
| Konsistenz | sehr hartes Gel * | sehr weiches Gel * | sehr weiches Gel ** | Gelteilchen in Flüssigkeit | flüssig |

*) und **) Bedeutung wie in Beispiel 12

## Beispiel 16

Beispiel 15 wurde für Versuch 7 aus Beispiel 14 wiederholt. Die Grenze der Gelbindung lag hier bei 27 % Polyurethanmatrix.

| Rezeptur (Teile) | | | | | |
|---|---|---|---|---|---|
| Polyol 6 | 75 | 75 | 75 | 75 | 75 |
| Polyol 4 | 25 | 65 | 75 | 90 | 100 |
| Polyisocyanat 2 | 7 | 7 | 7 | 7 | 7 |
| Dibutylzinndilaurat | 3 | 4,5 | 4,5 | 5 | 5 |
| % Polyurethanmatrix | 38 | 28 | 26 | 24 | 22 |
| Konsistenz | hartes Gel * | sehr weiches Gel * | sehr weiches Gel, teilweise flüssig | Gelteilchen in Flüssigkeit | flüssig |

*) erfindungsgemäß

## Beispiel 17

Für die Polyisocyanate 2, 3 und 4 wurde untersucht, welche Isocyanatkennzahl mindestens eingehalten werden muß, um bei der Reaktion mit verschiedenen Polyolen (unter Zusatz von 3 Gew.-% Methylbutyrat-Riechstoff) nach der Arbeitsweise von Beispiel 1 ein Gel zu erhalten : Die gefundenen Grenzwerte der Isocyanatkennzahl sind in der nachfolgenden Tabelle zusammengestellt.

| Polyisocyanat Nr./ | | | |
|---|---|---|---|
| Polyol Nr. | 2 | 4 | 3 |
| 8 | 20 | 30 | 32 |
| 15 | 18 | 35 | 37 |
| 11 | 30 | 45 | 47 |
| 3 | 25 | 55 | 60 |
| 9 | 25 | 55 | 65 |
| 10 | 32 | 65 | 70 |
| 12 | 40 | – | – |
| 13 | 50 | 50 | 52 |
| 16 | 20 | 50 | 52 |

### Beispiel 18

100 Teile Polyether 1 mit einer Temperatur von 70 °C, 25 Teile 2-Isopropoxyphenyl-N-methylcarbamat (Insektizid), 10 Teile 3-Phenoxy-4-fluor- -cyanobenzyl-2,2-dimethyl-3-/2-(4-chlorphenyl)-2-chlorvinyl/-cyclopropancarboxylat (Insektizid), 2,5 Teile Dibutylzinndilaurat und 5,5 Teile Polyisocyanat 2 werden intensiv vermischt. Das erhaltene Gel kann in Form von Platten, Streifen oder Formkörpern an Nutztieren, wie Rindern, in geeigneter Weise am Schwanz, Hals, an den Hörnern oder Ohren (Ohrmarken) befestigt werden. Auf diese Weise sind die Tiere wochenlang gegen zahlreiche schädliche tierische Parasiten (Ektoparasiten) geschützt.

### Beispiel 19

100 Teile Polyether 1, 15 Teile Diphenyl-acetylenyl-imidazolyl-methan (Algizid), 2 Teile Dibutylzinndilaurat und 5 Teile Polyisocyanat 2 werden intensiv vermischt. Nach 15 Minuten erhält man ein elastisches Gel. Ein derartiges Gel ist zur Beschichtung von z. B. Schiffen, Seetonnen oder Kaimauern im Unterwasserbereich geeignet, um den Bewuchs von Algen, Seepocken, Miesmuscheln und anderen Meereslebewesen zu verhindern.

### Beispiel 20

100 Teile Polyether 3, 5 Teile Menthol, 2,5 Teile Dibutylzinndilaurat und 8 Teile Polyisocyanat 2 werden intensiv vermischt. Die erhaltene Reaktionsmischung wird auf ein engmaschiges steifes Kunststoffgitter aus Polyethylen aufgegossen und erstarrt innerhalb von 30 Minuten zu einer elastischen Gelmasse. Ein derartiger Menthol-haltiger Strip kann für medizinische Zwecke (Inhalation von Menthol) verwendet werden.

### Beispiel 21

100 Teile Polyether, 1, 5 Teile Nonylphenol, 5 Teile Dodecylbenzyldimethylammonium-chlorid, 1,5 Teile Dibutylzinndilaurat und 5 Teile Polyisocyanat 2 werden intensiv vermischt. Das Reaktionsgemisch wird in eine offene Form mit den Abmessungen 1 × 2 × 10 cm gegossen. Der erhaltene Gelstab wird in eine 2 × 2 × 10 cm große Schale gelegt, die im Toilettenbecken in geeigneter Form derart befestigt wird, daß das Gel beim Wasserspülen jeweils stark gewässert wird. Auf diese Weise läßt sich eine längerfristige Desinfektion des Toilettenbeckens erzielen.

### Beispiel 22

100 Teile Polyether 3, 30 Teile Kaliumdichromat/Pentachlorphenyl (1 : 1), 3 Teile Dibutylzinndilaurat und 8 Teile Polyisocyanat 2 werden intensiv vermischt. Das erhaltene Reaktionsgemisch wird auf ein Polyestergewebe in 5 mm Stärke aufgetragen, auf welchem das Gemisch zu einem Gel erhärtet. Derartig beschichtete Polyestergewebe können in Form von Bandagen zum Umkleiden von Holzmasten im Übergangsbereich Erde/Luft verwendet werden, um das Holz gegen Fäulnis zu schützen.

### Beispiel 23

100 Teile Polyether 3, 15 Teile Natrium-dodecylbenzolsulfonat, 2,5 Teile Dibutylzinndilaurat und 8 Teile Polyisocyanat 2 werden intensiv vermischt und auf eine 10 mm starke offenzellige Folie aus Polyurethanschaum (Polyesterbasis) gesprüht. Eine derartig imprägnierte Folie, aufgeklebt auf einem Schwamm aus Polyether-Weichschaum, kann zu Reinigungszwecken verwendet werden.

### Beispiel 24

100 Teile Polyether 3 mit einer Temperatur von 40 °C, 15 Teile 1-Methyl-1-alkylamidoethyl-2-alkyl-imidazolinium-methosulfat (kationische quartäre Imidazolinverbindung der Fa. Ashland Chemical. Co., USA ; Wäscheweichmacher), 0,2 Teile Heliofast-Yellow C.I. n° 11680, 2 Teile Dibutylzinndilaurat und 8 Teile Polyisocyanat 2 werden intensiv vermischt. Das erhaltene Reaktionsgemisch wird in 3 mm starker Schicht auf ein Polypropylenvlies gegossen. Nach ca. 10 Minuten erhält man eine elastische Gelschicht. Das gelbeschichtete Vlies ist zum Weichmachen von Wäsche in Trommeltrocknern geeignet.

### Beispiel 25

a) Herstellung des Gels
100 Teile Polyether 1 und 5 Teile Polyisocyanat 2, sowie 1,5 Teile Dibutylzinndilaurat werden innerhalb von 1 Minute intensiv vermischt. Nach 10 Minuten erhält man ein trübes, elastisches Gel, das an seiner Oberfläche klebfrei ist.
b) Herstellung der Hohlform aus Gel
Das nach a) erhältliche gelfähige Gemisch kann innerhalb einer Zeit von 1-5 Minuten, gerechnet ab dem Vermischungsbeginn, zum Umgießen von z. B. einem Formteil aus Gips verwendet werden. Nach 15 Minuten, gerechnet ab dem Vermischungsbeginn, kann das Gipsmodell entnommen werden. Man erhält eine Gelform mit einem Hohlraum, dessen Volumen und Konturen denjenigen des entnommenen Gipsmodells entsprechen.

### Beispiel 26

a) Herstellung des Gels

> 10   Teile Polyether 1,
> 40   Teile Polyether 2,
> 50   Teile Polyether 3,
> 1,5 Teile Dibutylzinndilaurat und
> 6   Teile Polyisocyanat 2

werden innerhalb von 1 Minute intensiv vermischt. Nach 15 Minuten bildet sich ein klares, elastisches Gel, dessen Oberfläche klebfrei ist.
b) Herstellung der Hohlform aus Gel
Das nach a) erhältliche gelfähige Gemisch kann zum Abformen eines Formteils z. B. aus Epoxid verwendet werden. Nach ca. 20 Minuten kann das umgossene Epoxidmodell aus der Gelform entnommen werden. Die Gelform weist einen Hohlraum auf, der in den Konturen identisch mit denjenigen des Epoxidmodells ist.

### Beispiel 27

Analog zu Beispiel 26 werden ein Gel bzw. eine Hohlform hergestellt aus

> 10   Teilen Polyether 4,
> 50   Teilen Polyether 5,
> 40   Teilen Polyether 6 und
> 1,5 Teilen Dibutylzinndilaurat und
> 6   Teilen Polyisocyanat 2.

### Beispiel 28

> 3 500 Teile Polyether 3,
> 700 Teile Polyether 7 und
> 2 800 Teile Polyether 2

0 057 839

werden bei einer Temperatur von 22 °C mittels eines Labormischers mit Rührscheibe zu einer klaren Lösung verrührt. Zu dieser Lösung werden
301 Teile Polyisocyanat 2
unter Rühren zugegeben und gut verteilt. Zu der nun trüben Lösung werden
105 Teile Dibutylzinndilaurat
zugegeben und die Mischung 3 Minuten intensiv vermischt.

Die weißlich trübe Lösung wird in eine vorbereitete, quadratische Umhüllung aus Polyurethanfolie der Folienstärke 0,2 mm mit einer Kantenlänge von 45 cm gegossen und die Folienhülle luftdicht verschweißt. Das so vorgefertigte Gel-Polster wird auf eine ebene Unterlage gelegt und zur Gelreaktion sich selbst überlassen, wodurch das Gel-Polster seine mechanische Endfestigkeit erreicht und vollbelastet werden kann. Es ist ein weicher, formbeständiger, unter Druck deformierbarer Körper. Wird die deformierende Kraft aufgehoben, geht das Gel-Polster in seinen Ausgangszustand zurück.

Beispiel 29

3 500 Teile Polyether 3,
700 Teile Polyether 7,
2 800 Teile Polyether 2 und
35 Teile Dibutylzinndilaurat

werden in einem Rührkessel bei 22 °C homogen gemischt. Die Mischung wird mittels einer Zahnradpumpe einem statischen Mischer zugeführt. Aus einem getrennten Vorratsbehälter werden diesem Mischer mittels einer weiteren Zahnradpumpe gleichzeitig
473 Teile Polyisocyanat 2
so zugeführt, daß zu jeder Zeit das Mischungsverhältnis der beiden Komponenten gleich ist und dem Verhältnis der Gesamtmengen entspricht.

Die aus dem statischen Mischer ausfließende weißliche trübe Lösung wird in eine quadratische Umhüllung gegossen und daraus, wie in Beispiel 28 beschrieben, ein Gel-Polster in Form eines Kissens hergestellt.

Beispiel 30

1 000 Teile Polyether 1,
50 Teile Polyisocyanat 2 und
15 Teile Dibutylzinndilaurat

werden mit Hilfe eines Laborrührers mit Rührscheibe bei Raumtemperatur innerhalb von 1 Minute intensiv vermischt. Nach 10 Minuten erhält man ein trübes, elastisches, formstabiles Gel, das sich unter dem Einfluß einer darauf wirkenden Kraft leicht deformieren läßt und nach Aufheben der deformierenden Kraft seinen Ausgangszustand wieder einnimmt.

Beispiel 31

1 000 Teile Polyether 8,
25 Teile Polyisocyanat 3 und
30 Teile Dibutylzinndilaurat werden mit Hilfe eines Laborrührers mit einer Rührscheibe bei Raumtemperatur innerhalb von 1 Minute intensiv vermischt. Man erhält ein weiches, elastisches, formstabiles Gel, das sich unter dem Einfluß einer darauf wirkenden Kraft leicht deformieren läßt und nach Aufheben der deformierenden Kraft seinen Ausgangszustand wieder einnimmt.

Beispiel 32

1 000 Teile Polyether 8,
45 Teile Polyisocyanat 4 und
30 Teile Dibutylzinndilaurat werden mit Hilfe eines Laborrührers gemäß Beispiel 31 umgesetzt. Man erhält ein weiches, elastisches, formstabiles Gel, das sich unter dem Einfluß einer darauf wirkenden Kraft leicht deformieren läßt und nach Aufheben der deformierenden Kraft seinen Ausgangszustand wieder einnimmt.

17

### Beispiel 33

1 000 Teile Polyether 9, werden mit
50 Teilen Polyisocyanat 4 und
30 Teilen Dibutylzinndilaurat

analog Beispiel 31 zu einem weichen, elastischen, formstabilen Gel, das sich unter dem Einfluß einer darauf wirkenden Kraft leicht verformen läßt und nach Aufheben der deformierenden Kraft seinen Ausgangszustand wieder einnimmt, umgesetzt.

### Beispiel 34

Das Beispiel zeigt die erfindungsgemäße Mitverwendung von Weichmachungsmitteln.
490 Teile Polyether 3,
480 Teile Dibutyladipat,
30 Teile Polyisocyanat 2 und
15 Teile Dibutylzinndilaurat

werden gemäß Beispiel 31 zu einem weichen, elastischen, formstabilen Gel umgesetzt, das sich unter dem Einfluß einer darauf wirkenden Kraft leicht deformieren läßt und nach Aufheben der deformierenden Kraft seinen Ausgangszustand wieder einnimmt.

### Beispiel 35

Das Beispiel zeigt ebenfalls die erfindungsgemäße Mitverwendung von Weichmachungsmitteln.

508 Teile Polyether 3,
450 Teile eines Alkylsulfonsäureesters von Phenol,
27 Teile polyisocyanat 2 und
15 Teile Dibutylzinndilaurat

werden gemäß Beispiel 31 zu einem weichen, elastischen, formstabilen Gel umgesetzt, das sich unter dem Einfluß einer darauf wirkenden Kraft leicht deformieren läßt und nach Aufheben der deformierenden Kraft seinen Ausgangszustand wieder einnimmt.

### Beispiel 36

484 Teile Polyether 3,
450 Teile Alkylsulfonsäureester von Phenol,
51 Teile Polyisocyanat 4 und
15 Teile Dibutylzinndilaurat

werden gemäß Beispiel 31 zu einem weichen, elastischen, formstabilen Gel umgesetzt, das sich unter dem Einfluß einer darauf wirkenden Kraft leicht deformieren läßt und nach Aufheben der deformierenden Kraft seinen Ausgangszustand wieder einnimmt.

### Beispiel 37

Analog zu Beispiel 25 werden unter Variation der OH- bzw. NCO-Funktionalität der Ausgangskomponenten Gele hergestellt, wobei die Isocyanat-Kennzahl jeweils 50 betrug. Die Eigenschaften der so erhaltenen Gele sind in der nachfolgenden Tabelle zusammengestellt ; « flüssig » bedeutet, daß infolge zu niedriger Funktionalität noch keine Gelstruktur ausgebildet wurde.

Als Isocyanatkomponente wurden Polyisocyanat 1, Polyisocyanat 2 bzw. Gemische daraus mit der angegebenen mittleren NCO-Funktionalität verwendet ; die Polyolkomponente bestand aus den Polyolen 2, 10 bzw. 11 bzw. 1 : 1-Gemischen von 2 und 10 bzw. 10 und 11.

| Funktionalität: NCO \ OH | 2 | 2,3 | 2,75 | 3,25 | 4 |
|---|---|---|---|---|---|
| 2 | | | | flüssig | sehr weich |
| 2,1 | | | | sehr weich | weich |
| 2,2 | | | flüssig | weich· | hart |
| 2,3 | | | sehr weich | weich | |
| 2,4 | | | sehr weich | | |
| 2,6 | | flüssig | weich | | |
| 2,8 | | sehr weich | | | |
| 3,1 | | weich | | | |
| 3,6 | flüssig | hart | | | |

### Beispiel 38

Analog zu Beispiel 37 wurde die Abhängigkeit der Gelkonsistenz von der Funktionalität für die Isocyanat-Kennzahl 30 untersucht. Als Hydroxylkomponente wurden die Polyole 10, 11, 8 bzw. ein 1 : 1-Gemisch aus 11 und 8 verwendet.

| Funktionalität NCO \ OH | 2,75 | 4 | 4,8 | 6 |
|---|---|---|---|---|
| 2 | | | | flüssig |
| 2,1 | | | flüssig | sehr weich |
| 2,15 | | | flüssig | weich |
| 2,2 | | | sehr weich | weich-hart |
| 2,3 | | | sehr weich | hart |
| 2,4 | | flüssig | weich | hart |
| 2,8 | | sehr weich | weich | hart |
| 3,6 | sehr weich | weich | | |

### Beispiel 39

In Analogie zu Beispiel 37 wurde die Abhängigkeit der Gelkonsistenz von Isocyanatkennzahl und NCO-Funtkionalität untersucht. Als Polyolkomponente wurden ein 1 : 1-Gemisch der Polyole 2 und 12 eingesetzt, als Isocyanatkomponente Gemische der Polyisocyanate 1 und 2 mit der angegebenen mittleren NCO-Funktionalität.

| Funktionalität NCO \ Kennzahl | 2,6 | 2,8 | 3,0 | 3,2 |
|---|---|---|---|---|
| 55 | sehr weich | | | |
| 52,5 | flüssig | | | |
| 50 | flüssig | weich | | |
| 47,5 | flüssig | sehr weich | weich | hart |

19

# 0 057 839

Beispiel 40

Abhängigkeit der Gelkonsistenz von der NCO-Funktionalität bei konstanter Isocyanatkennzahl (50) und OH-Funktionalität (3).

Versuch 1
Polyolkomponente. Polyol 6
Isocyanatkomponente : Gemische aus Polyisocyanaten 1 und 2
Versuch 2
Polyolkomponente : Polyol 4/Polyol 6 (1 : 1)
Isocyanatkomponente : wie Versuch 1

| NCO-Funktionalität | Versuch 1 | Versuch 2 |
|---|---|---|
| 2 | flüssig | flüssig |
| 2,1 | flüssig | sehr weich - weich |
| 2,2 | sehr weich | weich |
| 2,3 | weich | weich - hart |
| 2,4 | weich - hart | hart |
| 2,6 | hart | hart |
| 2,8 | hart | sehr hart |

Beispiel 41

Abhängigkeit der Gelkonsistenz vom Mischungsverhältnis Polyether mit primären Hydroxylgruppen/Polyether mit sekundären Hydroxylgruppen.

Isocyanatkennzahl : 35
Isocyanatkomponente : Polyisocyanat 2
Die Gele werden analog zu Beispiel 25 hergestellt.

| Versuch | Polyol 6 (%) | Polyol 4 (%) | Gelkonsistenz |
|---|---|---|---|
| 1 | 0 | 100 | sehr weich |
| 2 | 5 | 95 | weich |
| 3 | 15 | 85 | weich-hart |
| 4 | 25 | 75 | hart |
| 5 | 35 | 65 | sehr hart |
| 6 | 45 | 55 | hart |
| 7 | 75 | 25 | hart |
| 8 | 100 | 0 | weich-hart |

Beispiel 42

Für Versuch 5 von Beispiel 41 wurde untersucht, wieviel des (praktisch nicht mitreagierenden) Polyols 4 bei sonst gleicher Rezeptur dem Reaktionsansatz zugesetzt werden kann, so daß noch ein Gel erhalten wird. Wie die nachfolgende Tabelle zeigt, liegt die Grenze der Gelbildung für die gewählten Ausgangskomponenten etwa bei einer Zusammensetzung, die (theoretisch berechnet) 28 % Polyurethanmatrix und 72 % freiem Polyol entspricht.

(Siehe Tabelle Seite 21 f.)

20

| Rezeptur (Teile) | | | | | |
|---|---|---|---|---|---|
| Polyol 6 | 35 | 35 | 35 | 35 | 35 |
| Polyol 4 | 65 | 100 | 105 | 120 | 150 |
| Polyisocy-anat 2 | 7 | 7 | 7 | 7 | 7 |
| Dibutylzinn-dilaurat | 3 | 3 | 3 | 4 | 5 |
| % Polyurethan-matrix | 38 | 29 | 28 | 25 | 21 |
| Konsistenz | sehr har-tes Gel | sehr wei-ches Gel | sehr wei-ches Gel | Gelteil-chen in Flüssig-keit | flüssig |

Beispiel 43

Beispiel 42 wurde wiederholt für Versuch 7 aus Beispiel 41. Die Grenze der Gelbildung lag hier bei ca. 27 % Polyurethanmatrix.

| Rezeptur (Teile) | | | | | |
|---|---|---|---|---|---|
| Polyol 6 | 75 | 75 | 75 | 75 | 75 |
| Polyol 4 | 25 | 65 | 75 | 90 | 100 |
| Polyisocya-nat 2 | 7 | 7 | 7 | 7 | 7 |
| Dibutylzinn-dilaurat | 3 | 4,5 | 4,5 | 5 | 5 |
| % Polyurethan-matrix | 38 | 28 | 26 | 24 | 22 |
| Konsistenz | hartes Gel | sehr wei-ches Gel | sehr wei-ches Gel, teilweise flüssig | Gelteil-chen in Flüssig-keit | flüssig |

Beispiel 44

Für die Polyisocyanate 2, 3 und 4 wurde untersucht, welche Isocyanatkennzahl mindestens eingehalten werden muß, um bei der Reaktion mit verschiedenen Polyolen nach der Arbeitsweise von Beispiel 25 ein Gel zu erhalten. Die gefundenen Grenzwerte der Isocyanatkennzahl sind in der nachfolgenden Tabelle zusammengestellt.

| Polyisocyanat Nr.<br>Polyol Nr. | 2 | 4 | 3 |
|---|---|---|---|
| 8 | 20 | 30 | 32 |
| 15 | 18 | 35 | 37 |
| 11 | 30 | 45 | 47 |
| 3 | 25 | 55 | 60 |
| 9 | 25 | 55 | 65 |
| 10 | 32 | 65 | 70 |
| 12 | 40 | – | – |
| 13 | 50 | – | – |
| 16 | 20 | 50 | 52 |

Beispiel 45

In Analogie zu den Beispielen 25 und 26 wurden mit den in der nachstehenden Tabelle angegebenen Rezepturen Gele und Abformmassen hergestellt. Der verwendete Weichmacher war Dibutyladipat ; der Katalysator Dibutylzinndilaurat.

(Siehe Tabelle Seite 23 f.)

Rezeptur (Teile)

| | | | | | | | | |
|---|---|---|---|---|---|---|---|---|
| Polyol 14 | 100 | 10 | 80 | | | | | |
| Polyol 3 | | 50 | | 100 | 100 | 50,5 | 100 | 50,5 |
| Polyol 2 | | 40 | | | | | | |
| Polyol 16 | | | 20 | | | | | |
| Weichmacher | | | | | | 45 | | |
| Kaolin | | | | | | | 107,5 | |
| o-Dichlorbenzol | | | | | | | | 45 |
| Katalysator | 1,5 | 1,5 | 1,5 | 1,5 | 1,5 | 1,5 | 1,5 | 1,5 |
| Polyisocyanat 2 | 5 | 5,5 | 7,3 | 2 | | 3 | 6 | 3 |
| Polyisocyanat 5 | | | | | 50 | | | |
| Polyisocyanat 4 | | | | 6 | | | | |

0 057 839

## 0 057 839

**Patentansprüche**

1. Gele, bestehend aus
(1) 15-62 Gew.-%, bezogen auf die Summe aus (1) und (2), einer hochmolekularen Matrix und
(2) 85-38 Gew.-%, bezogen auf die Summe aus (1) und (2), eines in der Matrix durch Nebenvalenzkräfte fest gebundene flüssigen Dispersionsmittels, sowie gegebenenfalls
(3) 0-100 Gew.-%, bezogen auf die Summe aus (1) und (2), an Füll- und/oder Zusatzstoffen, sowie gegebenenfalls Katalysatoren für die Isocyanat-Polyadditionsreaktion,
dadurch gekennzeichnet, daß
a) die hochmolekulare Matrix ein kovalent vernetztes Polyurethan ist und
b) das flüssige Dispersionsmittel aus einer oder mehreren Polyhydroxylverbindungen mit einem Molekulargewicht zwischen 1 000 und 12 000 und einer OH-Zahl zwischen 20 und 112 besteht, wobei das Dispersionsmittel im wesentlichen keine Hydroxylverbindungen mit einem Molekulargewicht unter 800 enthält,
und wobei das Produkt der Funktionalitäten der polyurethanbildenden Komponenten mindestens 5,2 beträgt und die Isocyanatkennzahl zwischen 15 und 60 liegt.

2. Gele nach Anspruch 1, dadurch gekennzeichnet, daß sie aus 20-57 Gew.-% der hochmolekularen Matrix und 80-43 Gew.-% des flüssigen Dispersionsmittels bestehen, und daß die hochmolekulare Matrix ein Umsetzungsprodukt aus einem oder mehreren Polyisocyanaten und einer oder mehreren Polyhydroxylverbindungen mit einem Molekulargewicht zwischen 1 000 und 12 000 und einer OH-Zahl zwischen 20 und 112 ist, wobei das Produkt aus NCO-Funktionalität der Polyisocyanate und OH-Funktionalität der Polyhydroxylverbindungen mindestens 5,2 beträgt.

3. Gele nach Ansprüchen 1 und 2, dadurch gekennzeichnet, daß 0,1 bis 50 Gew.-% an Wirkstoffen als Zusatzstoffe in der wirkstoffhaltigen Gelmasse enthalten sind.

4. Gele nach Ansprüchen 1 bis 3, dadurch gekennzeichnet, daß Wirkstoffe aus der Gruppe der Biozide, Duftstoffe, Farben, Detergentien und Waschhilfsmittel, Stempel- und Druckfarben, Alterungsschutzmittel, Gleitmittel und Antistatika, Reinigungs- und Pflegemittel, Antifoulingmittel und Holzschutzmittel, sowie Pflanzennährstoffe, Frischhaltemittel und Wachstumsregulatoren enthalten sind.

5. Verfahren zur Herstellung von gegebenenfalls wirkstoffhaltigen, wasserfreien Gelmassen auf der Basis von Polyurethangelen, dadurch gekennzeichnet, daß man
a) ein oder mehrere Di- und/oder Polyisocyanate mit
b) einer oder mehreren polyhydroxylverbindungen mit einem Molekulargewicht zwischen 1 000 und 12 000 und einer OH-Zahl zwischen 20 und 112,
gegebenenfalls
c) 0,1 bis 50 Gew.-% an Wirkstoffen,
gegebenenfalls
d) Katalysatoren für die Reaktion zwischen isocyanat- und Hydroxylgruppen,
sowie gegebenenfalls
e) aus der Polyurethanchemie an sich bekannten Füll- und Zusatzstoffen
umsetzt, wobei die Isocyanatkennzahl zwischen 15 und 60 liegt,
das Produkt der Funktionalitäten der Polyurethanbildenden Komponenten mindestens 5,2 beträgt und die Polyhydroxylverbindungen im wesentlichen frei an Hydroxylverbindungen mit einem Molekulargewicht unter 800 sind.

6. Verfahren nach Anspruch 5, dadurch gekennzeichnet, daß die Polyhydroxylverbindungen ein Molekulargewicht zwischen 1 700 und 6 000 aufweisen, das Produkt der Funktionalitäten der polyurethanbildenden Komponenten mindestens 6,2 beträgt und daß gegebenenfalls 0,5 bis 35 Gew.-% an Wirkstoffen in Polyolverbindungen gelöst oder dispergiert verwendet werden.

7. Verfahren nach Ansprüchen 1 bis 6, dadurch gekennzeichnet, daß Wirkstoffe aus der Gruppe der Biozide, Pharmazeutika, Naturstoffe wie etherische Öle, Duftstoffe, Farben, Detergentien und Waschhilfsmittel, Stempel- und Druckfarben, Alterungsschutzmittel, Gleitmittel und Antistatika, Reinigungs- und Pflegemittel, Antifoulingmittel und Holzschutzmittel, sowie Pflanzennährstoffe, Frischhaltemittel und Wachstumsregulatoren verwendet werden, welche frei von reaktiven Gruppen sind, die unter den Bedingungen der Gelbildung weitgehend oder vollständig unter fixierung reagieren.

8. Verwendung von Gelen nach Ansprüchen 1 bis 7 als Abformmaterialien und Eingußmassen.

9. Verwendung von Gelen nach Anspruch 8 zum Abformen von Gegenständen durch Umgießen des abzuformenden Körpers mit einer gelbildenden Masse und Entnahme des Formkörpers nach der Gelbildung, dadurch gekennzeichnet, daß man den Körper mit einer Mischung aus
a) einem oder mehreren Polyisocyanaten,
b) einer oder mehreren Polyhydroxylverbindungen mit einem Molekulargewicht zwischen 1 000 und 12 000, und einer OH-Zahl zwischen 20 und 112,
c) gegebenenfalls Katalysatoren für die Reaktion zwischen Isocyanat- und Hydroxylgruppen sowie gegebenenfalls
d) aus der Polyurethanchemie an sich bekannten Füll- und Zusatzstoffen
gegebenenfalls in mehreren Schichten mit gegebenenfalls unterschiedlicher Zusammensetzung umgießt, wobei

24

diese Mischung im wesentlichen frei ist an Hydroxylverbindungen mit einem Molekulargewicht unter 800,

die Isocyanatkennzahl zwischen 15 und 60 liegt

und das Produkt der Funktionalität der polyurethanbildenen Komponenten mindestens 5,2 beträgt, die Mischung gelieren läßt und entformt.

10. Verwendung von wirkstoffhaltigen Gelen nach Anspruch 4, als Wirkstoffträger mit Depotwirkung, dadurch gekennzeichnet, daß sie zur längerfristigen Abgabe der Wirkstoffe aus den Gelformteilen in Form von

duftstoffhaltigen Formteilen zur Beduftung von Räumen,

in Form von insektizidhaltigen Formteilen zur Bekämpfung von Fliegen und Ungeziefer,

in Form von desodorisierenden Formteilen zur Übertragung auf die Haut,

in Form von dermatologische Wirkstoffe enthaltenden Pflastern oder

in Form von Druck- und Stempelplatten geringer Trocknungstendenz

eingesetzt werden.

## Claims

1. Gels consisting of

(1) 15-62 % by weight, based on the sum of (1) and (2), of a high molecular weight matrix, and

(2) 85-38 % by weight, based on the sum of (1) and (2), of a liquid dispersing agent firmly bound in the matrix by secondary valency forces, and optionally

(3) 0-100 % by weight, based on the sum of (1) and (2), of fillers and/or additives and optionally catalysts for the isocyanate polyaddition reaction,

characterised in that

a) the high molecular weight matrix is a covalently crosslinked polyurethane and

b) the liquid dispersing agent consists of one or more polyhydroxyl compounds having a molecular weight between 1 000 and 12 000 and an OH number between 20 and 112, the dispersing agent being substantially free from any hydroxyl compounds having a molecular weight below 800, and the product of the functionalities of the polyurethane-forming components is at least 5.2 and the isocyanate index is between 15 and 60.

2. Gels according to claim 1, characterised in that they consist of 20 to 57 % by weight of the high molecular weight matrix and 80 to 43 % by weight of the liquid dispersing agent and in that the high molecular weight matrix is a reaction product of one or more polyisocyanates and one or more polyhydroxyl compounds having a molecular weight between 1 000 and 12 000 and an OH number between 20 and 112, the product of NCO functionality of the polyisocyanates and OH functionality of the polyhydroxyl compounds being at least 5.2.

3. Gels according to claims 1 and 2, characterised in that 0.1 to 50 % by weight of active ingredients are contained as additives in the gel composition containing active ingredients.

4. Gels according to claims 1 to 3, characterised in that they contain active ingredients from the group comprising biocidal agents, perfume substances, dyes, detergents and washing aids, stamping and printing inks, age resistors, lubricants and antistatic agents, cleaning and polishing agents, antifouling agents and wood preservatives, as well as plant nutrients, preserving agents and growth regulators.

5. Process for the preparation of anhydrous gel compositions based on polyurethane gels and optionally containing active ingredients, characterised in that

a) one or more di- and/or polyisocyanates are reacted with

b) one or more polyhydroxyl compounds having a molecular weight between 1 000 and 12 000 and an OH number between 20 and 112, optionally

c) 0.1 to 50 % by weight of active ingredients, optionally

d) catalysts for the reaction between isocyanate and hydroxyl groups, and optionally

e) fillers and additives known in polyurethane chemistry,

the isocyanate index being between 15 and 60, the product of the functionalities of the polyurethane-forming components being at least 5.2, and the polyhydroxyl compounds being substantially free from hydroxyl compounds having a molecular weight below 800.

6. Process according to claim 5, characterised in that the polyhydroxyl compounds have a molecular weight between 1 700 and 6 000 and the product of the functionalities of the polyurethane-forming components is at least 6.2, and in that 0.5 to 35 % by weight of active ingredients dissolved or dispersed in polyol compounds are optionally used.

7. Process according to claims 1 to 6, characterised in that active ingredients from the group comprising biocidal agents, pharmaceuticals, natural substances such as ethereal oils, perfume ingredients, dyes, detergents and washing aids, stamping and printing inks, age resistors, lubricants and antistatic agents, cleaning and polishing agents, antifouling agents and wood preservatives, as well as plant nutrients, preserving agents and growth regulators are used, which substances are free from reactive groups which under the conditions of gel formation react to become extensively or completely chemically fixed.

8. Use of gels according to claims 1 to 7 as replication materials and casting compositions.

9. Use of gels according to claim 8 for replicating articles by pouring a gel-forming composition over the body which is to be replicated and removing the body after gel formation, characterised in that a mixture of

a) one or more polyisocyanates,

b) one or more polyhydroxyl compounds having a molecular weight between 1 000 and 12 000 and an OH number between 20 and 112,

c) optionally catalysts for the reaction between isocyanate groups and hydroxyl groups and optionnaly

d) fillers and additives known from polyurethane chemistry is poured over the body optionally in several layers optionally differing in composition,

the said mixture being substantially free from hydroxyl compounds having a molecular weight below 800,

the isocyanate index being between 15 and 60,

and the product of the functionality of the polyurethane-forming components being at least 5.2,

and the mixture is left to gel and the body is removed from the mould.

10. Use of gels containing active ingredients according to claim 4 as active ingredient carriers with a depot action, characterised in that they are used for the long term release of active ingredients from the shaped gel products in the form

of mouldings containing perfume ingredients for scenting rooms, in the form

of mouldings containing insecticides for controlling flies and vermin, in the form

of deodorant mouldings for transfer to the skin, in the form

of plasters containing dermatologically active ingredients, or in the form

of printing and stamping plates with little tendency to dry out.

## Revendications

1. Gels, constitués par

(1) 15-62 % en poids, par rapport à la somme de (1) et (2), d'une matrice de haut poids moléculaire et

(2) 85-38 % en poids, par rapport à la somme de (1) et (2), d'un agent dispersant liquide solidement fixé à la matrice par des liaisons de covalence, ainsi que le cas échéant

(3) 0-100 % en poids, par rapport à la somme de (1) et (2), de charges et/ou d'additifs, ainsi que, le cas échéant, de catalyseurs pour la réaction de polyaddition d'un isocyanate, caractérisés en ce que

a) la matrice de haut poids moléculaire est un polyuréthanne réticulé par covalence et

b) l'agent dispersant liquide est formé d'un ou plusieurs composés polyhydroxyliques ayant un poids moléculaire compris entre 1 000 et 12 000 et un indice d'hydroxyle de 20 à 112, l'agent dispersant ne contenant essentiellement pas de composés hydroxyliques ayant un poids moléculaire inférieur à 800, et le produit des fonctionnalités des composants formant le polyuréthanne s'élève alors au moins à 5,2 et l'indice d'isocyanate se situe entre 15 et 60.

2. Gels suivant la revendication 1, caractérisés en ce qu'ils sont formés de 20 à 57 % en poids de la matrice de haut poids moléculaire et de 80 à 43 % en poids de l'agent dispersant liquide, et en ce que la matrice de haut poids moléculaire est un produit de réaction d'un ou plusieurs poly-isocyanates et d'un ou plusieurs composés polyhydroxyliques ayant un poids moléculaire de 1 000 à 12 000 et un indice d'hydroxyle de 20 à 112, le produit de la fonctionnalité NCO des polyisocyanates et de la fonctionnalité OH des composés polyhydroxyliques s'élevant au moins à 5,2.

3. Gels suivant les revendications 1 et 2, caractérisés en ce qu'une proportion de 0,1 à 50 % en poids de substances actives est contenue comme additifs dans la matière gélifiée contenant une substance active.

4. Gels suivant les revendications 1 à 3, caractérisés en ce qu'ils contiennent des substances actives du groupe des biocides, des parfums, des colorants, des détergents et produits pour la lessive, des encres pour tampons et encres d'impression, des agents de protection contre le vieillissement, des lubrifiants et des antistatiques, des produits de nettoyage et des produits d'entretien, des agents antifouling et des agents de protection du bois ainsi que des substances nutritives pour les plantes, des préservateurs et des substances influençant la croissance.

5. Procédé de production de matières gélifiées anhydres contenant éventuellement des substances actives, à base de gels de polyuréthanne, caractérisé en ce qu'on fait réagir

a) un ou plusieurs di- et/ou poly-isocyanates avec

b) un ou plusieurs composés polyhydroxyliques ayant un poids moléculaire de 1 000 à 12 000 et un indice d'hydroxyle de 20 à 122,

le cas échéant

     c) 0,1 à 50 % en poids de substances actives, le cas échéant

     d) des catalyseurs pour la réaction entre groupes isocyanate et hydroxyle, ainsi que, le cas échéant,

     e) des charges et des additifs connus dans la chimie des polyuréthannes, l'indice d'isocyanate étant compris entre 15 et 60,

le produit des fonctionnalités des composants formant le polyuréthanne s'élevant à 5,2 au moins et les composés polyhydroxyliques étant essentiellement dépourvus de composés hydroxyliques ayant un poids moléculaire inférieur à 800.

     6. Procédé suivant la revendication 5, caractérisé en ce que les composés polyhydroxyliques ont un poids moléculaire compris entre 1 700 et 6 000, le produit des fonctionnalités des composants formant le polyuréthanne s'élève au moins à 6,2 et on utilise éventuellement de 0,5 à 35 % en poids de substances actives dissoutes ou dispersées dans des composés qui sont des polyols.

     7. Procédé suivant les revendications 1 à 6, caractérisé en ce qu'on utilise des substances actives du groupe des biocides, des substances pharmaceutiques, des substances naturelles telles que des huiles essentielles, des parfums, des colorants, des détergents et des produits pour la lessive, des encres pour tampons et des encres d'impression des agents de protection contre le vieillissement, des lubrifiants et des antistatiques, des produits de nettoyage et d'entretien, des agents antifouling et des agents de protection du bois ainsi que des substances nutritives pour les plantes, des préservateurs et des substances de croissance, qui sont dépourvus de groupes réactifs qui, dans les conditions de la formation du gel, réagissent largement ou totalement en se fixant.

     8. Utilisation de gels suivant les revendications 1 à 7 comme matières de moulage et mélanges à couler.

     9. Utilisation de gels suivant la revendication 8 pour mouler des objets en entourant le corps à mouler d'une matière formant un gel et en retirant le corps moulé après la formation du gel, caractérisée en ce qu'on entoure le corps d'un mélange formé

     a) d'un ou plusieurs composés poly-isocyanate,

     b) d'un ou plusieurs composés polyhydroxyliques ayant un poids moléculaire entre 1 000 et 12 000 et un indice d'hydroxyle entre 20 et 112,

     c) éventuellement des catalyseurs pour la réaction entre des groupes isocyanate et des groupes hydroxyle ainsi que le cas échéant.

     d) des charges et des additifs connus dans la chimie des polyuréthannes éventuellement en plusieurs couches ayant le cas échéant une composition différente,

ce mélange étant essentiellement dépourvu de composés hydroxyliques ayant un poids moléculaire inférieur à 800,

l'indice d'isocyanate étant compris entre 15 et 60

et le produit de la fonctionnalité des composants formant le polyuréthanne s'élevant au moins à 5,2, on laisse le mélange se gélifier et on démoule.

     10. Utilisation de gels contenant une substance active suivant la revendication 4 comme support de substance active à action retardée, caractérisée en ce qu'on utilise des gels pour la libération prolongée des substances actives des pièces moulées gélifiées sous la forme

de pièces moulées contenant un parfum pour parfumer des locaux,

de pièces moulées contenant un insecticide pour combattre des mouches et des insectes parasites,

de pièces moulées désodorisantes pour l'application à la peau,

d'emplâtres contenant des substances actives dermatologiques ou,

de plaques d'impression et de timbrage ayant peu tendance à sécher.